# EUROPEAN PATENT APPLICATION

(11) **EP 3 381 946 A1**
(43) Date of publication of application: **03.10.2018**
(21) Application number: 16867848.0
(22) Date of filing: 26.10.2016
(51) Int. Cl.: C07K 19/00, A61K 39/00, A61P 35/00

(54) **ANTI-PLACENTA-CHONDROITIN-SULFATE CHIMERIC ANTIGEN RECEPTOR AND APPLICATION THEREOF**

(30) Priority: 26.11.2015 CN 201510843720; 18.01.2016 CN 201610032290
(71) Applicant: Guangzhou Cas Lamvac Biotech Co., Ltd., Guangzhou, Guangdong 510530 (CN)
(72) Inventor: YAO, Yongchao, Guangzhou Guangdong 510530 (CN); QI, Hemei, Guangzhou Guangdong 510530 (CN); GUO, Wenzhong, Guangzhou Guangdong 510530 (CN); QIN, Li, Guangzhou Guangdong 510530 (CN); CHEN, Xiaoping, Guangzhou Guangdong 510530 (CN)
(74) Representative: HGF Limited
(86) International application number: PCT/CN2016/103457
(87) International publication number: WO 2017/088623

(57) **Abstract**

The present invention provides an anti-placenta chondroitin sulfate (pl-CS) chimeric antigen receptor and application thereof. The chimeric antigen receptor mainly comprises anti-pl-CS antigen recognition region, a hinge region, a transmembrane region and intracellular region, wherein the anti-pl-CS antigen recognition region is any one of a plasmodium protein VAR2CSA, a part of peptide segment of plasmodium protein VAR2CSA or a pl-CS antibody; and the part of peptide segment of the plasmodium protein VAR2CSA is a peptide segment with the number of amino acid greater than 500 in the plasmodium protein VAR2CSA.

SEQUENCE LISTING

<110>  GUANGZHOU CAS LAMVAC BIOTECH CO., LTD

<120>  ANTI-PLACENTA-CHONDROITIN-SULFATE CHIMERIC ANTIGEN RECEPTOR AND
APPLICATION THEREOF

<130>  2015

<140> PCT/CN2016/103457
<141> 2016-10-26

<160>  22

<170>  PatentIn version 3.3

<210>  1
<211>  900
<212>  PRT
<213>  artificial sequence

<220>
<223> amino acid sequence of a chimeric antigen receptor

<400>  1

Asn Tyr Ile Lys Gly Asp Pro Tyr Phe Ala Glu Tyr Ala Thr Lys Leu
1               5                   10                  15

Ser Phe Ile Leu Asn Ser Ser Asp Ala Asn Asn Pro Ser Glu Lys Ile
            20                  25                  30

Gln Lys Asn Asn Asp Glu Val Cys Asn Cys Asn Glu Ser Gly Ile Ala
        35                  40                  45

Ser Val Glu Gln Glu Gln Ile Ser Asp Pro Ser Ser Asn Lys Thr Cys
    50                  55                  60

Ile Thr His Ser Ser Ile Lys Ala Asn Lys Lys Lys Val Cys Lys His
65                  70                  75                  80

Val Lys Leu Gly Val Arg Glu Asn Asp Lys Asp Leu Arg Val Cys Val
                85                  90                  95

Ile Glu His Thr Ser Leu Ser Gly Val Glu Asn Cys Cys Cys Gln Asp
            100                 105                 110

Phe Leu Arg Ile Leu Gln Glu Asn Cys Ser Asp Asn Lys Ser Gly Ser
        115                 120                 125

Ser Ser Asn Gly Ser Cys Asn Asn Lys Asn Gln Glu Ala Cys Glu Lys
    130                 135                 140

Asn Leu Glu Lys Val Leu Ala Ser Leu Thr Asn Cys Tyr Lys Cys Asp

145                 150                 155                 160

Lys Cys Lys Ser Glu Gln Ser Lys Lys Asn Asn Lys Asn Trp Ile Trp
                165                 170                 175

Lys Lys Ser Ser Gly Lys Glu Gly Gly Leu Gln Lys Glu Tyr Ala Asn
            180                 185                 190

Thr Ile Gly Leu Pro Pro Arg Thr Gln Ser Leu Cys Leu Val Val Cys
        195                 200                 205

Leu Asp Glu Lys Gly Lys Lys Thr Gln Glu Leu Lys Asn Ile Arg Thr
    210                 215                 220

Asn Ser Glu Leu Leu Lys Glu Trp Ile Ile Ala Ala Phe His Glu Gly
225                 230                 235                 240

Lys Asn Leu Lys Pro Ser His Glu Lys Lys Asn Asp Asp Asn Gly Lys
                245                 250                 255

Lys Leu Cys Lys Ala Leu Glu Tyr Ser Phe Ala Asp Tyr Gly Asp Leu
            260                 265                 270

Ile Lys Gly Thr Ser Ile Trp Asp Asn Glu Tyr Thr Lys Asp Leu Glu
        275                 280                 285

Leu Asn Leu Gln Lys Ile Phe Gly Lys Leu Phe Arg Lys Tyr Ile Lys
    290                 295                 300

Lys Asn Asn Thr Ala Glu Gln Asp Thr Ser Tyr Ser Ser Leu Asp Glu
305                 310                 315                 320

Leu Arg Glu Ser Trp Trp Asn Thr Asn Lys Lys Tyr Ile Trp Leu Ala
                325                 330                 335

Met Lys His Gly Ala Gly Met Asn Ser Thr Thr Cys Cys Gly Asp Gly
            340                 345                 350

Ser Val Thr Gly Ser Gly Ser Ser Cys Asp Asp Ile Pro Thr Ile Asp
        355                 360                 365

Leu Ile Pro Gln Tyr Leu Arg Phe Leu Gln Glu Trp Val Glu His Phe
    370                 375                 380

Cys Lys Gln Arg Gln Glu Lys Val Lys Pro Val Ile Glu Asn Cys Lys
385                 390                 395                 400

Ser Cys Lys Glu Ser Gly Gly Thr Cys Asn Gly Glu Cys Lys Thr Glu
                405                 410                 415

Cys Lys Asn Lys Cys Glu Val Tyr Lys Lys Phe Ile Glu Asp Cys Lys
            420                 425                 430

Gly Gly Asp Gly Thr Ala Gly Ser Ser Trp Val Lys Arg Trp Asp Gln
        435                 440                 445

Ile Tyr Lys Arg Tyr Ser Lys Tyr Ile Glu Asp Ala Lys Arg Asn Arg
    450                 455                 460

Lys Ala Gly Thr Lys Asn Cys Gly Pro Ser Ser Thr Thr Asn Ala Ala
465                 470                 475                 480

Glu Asn Lys Cys Val Gln Ser Asp Ile Asp Ser Phe Phe Lys His Leu
                485                 490                 495

Ile Asp Ile Gly Leu Thr Thr Pro Ser Ser Tyr Leu Ser Ile Val Leu
            500                 505                 510

Asp Asp Asn Ile Cys Gly Ala Asp Lys Ala Pro Trp Thr Thr Tyr Thr
        515                 520                 525

Thr Tyr Thr Thr Thr Glu Lys Cys Asn Lys Glu Thr Asp Lys Ser Lys
    530                 535                 540

Leu Gln Gln Cys Asn Thr Ala Val Val Val Asn Val Pro Ser Pro Leu
545                 550                 555                 560

Gly Asn Thr Pro His Gly Tyr Lys Tyr Ala Cys Gln Cys Lys Ile Pro
                565                 570                 575

Thr Asn Glu Glu Thr Cys Asp Asp Arg Lys Glu Tyr Met Asn Gln Trp
            580                 585                 590

Ser Cys Gly Ser Ala Arg Thr Met Lys Arg Gly Tyr Lys Asn Asp Asn
        595                 600                 605

Tyr Glu Leu Cys Lys Tyr Asn Gly Val Asp Val Lys Pro Thr Thr Val
    610                 615                 620

Arg Ser Asn Ser Ser Lys Leu Asp Thr Thr Thr Pro Ala Pro Arg Pro
625                 630                 635                 640

Pro Thr Pro Ala Pro Thr Ile Ala Ser Gln Pro Leu Ser Leu Arg Pro
                645                 650                 655

Glu Ala Cys Arg Pro Ala Ala Gly Gly Ala Val His Thr Arg Gly Leu
            660                 665                 670

Asp Phe Ala Cys Asp Phe Trp Val Leu Val Val Val Gly Gly Val Leu
        675                 680                 685

Ala Cys Tyr Ser Leu Leu Val Thr Val Ala Phe Ile Ile Phe Trp Val
    690                 695                 700

Arg Ser Lys Arg Ser Arg Leu Leu His Ser Asp Tyr Met Asn Met Thr
705                 710                 715                 720

Pro Arg Arg Pro Gly Pro Thr Arg Lys His Tyr Gln Pro Tyr Ala Pro
                725                 730                 735

Pro Arg Asp Phe Ala Ala Tyr Arg Ser Lys Arg Gly Arg Lys Lys Leu
            740                 745                 750

Leu Tyr Ile Phe Lys Gln Pro Phe Met Arg Pro Val Gln Thr Thr Gln
        755                 760                 765

Glu Glu Asp Gly Cys Ser Cys Arg Phe Pro Glu Glu Glu Glu Gly Gly
    770                 775                 780

Cys Glu Leu Arg Val Lys Phe Ser Arg Ser Ala Asp Ala Pro Ala Tyr
785                 790                 795                 800

Gln Gln Gly Gln Asn Gln Leu Tyr Asn Glu Leu Asn Leu Gly Arg Arg
                805                 810                 815

Glu Glu Tyr Asp Val Leu Asp Lys Arg Arg Gly Arg Asp Pro Glu Met
            820                 825                 830

Gly Gly Lys Pro Gln Arg Arg Lys Asn Pro Gln Glu Gly Leu Tyr Asn
        835                 840                 845

Glu Leu Gln Lys Asp Lys Met Ala Glu Ala Tyr Ser Glu Ile Gly Met
    850                 855                 860

Lys Gly Glu Arg Arg Arg Gly Lys Gly His Asp Gly Leu Tyr Gln Gly
865                 870                 875                 880

Leu Ser Thr Ala Thr Lys Asp Thr Tyr Asp Ala Leu His Met Gln Ala
                885                 890                 895

Leu Pro Pro Arg
            900

<210>  2
<211>  2700
<212>  DNA
<213>  artificial sequence

<220>
<223>  nucleotide sequence encoding a chimeric antigen receptor

<400>  2
aactacatca agggcgaccc ctacttcgcc gagtacgcca ccaagctgag cttcatcctg      60

aacagcagcg acgccaacaa ccccagcgag aagatccaga agaacaacga cgaggtgtgc     120

aactgcaacg agagcggcat cgccagcgtg gagcaggagc agatcagcga ccccagcagc     180

aacaagacct gcatcaccca cagcagcatc aaggccaaca agaagaaggt gtgcaagcac     240

gtgaagctgg gcgtgcgcga gaacgacaag gacctgcgcg tgtgcgtgat cgagcacacc     300

agcctgagcg gcgtggagaa ctgctgctgc caggacttcc tgcgcatcct gcaggagaac     360

tgcagcgaca acaagagcgg cagcagcagc aacggcagct gcaacaacaa gaaccaggag     420

gcctgcgaga agaacctgga gaaggtgctg gccagcctga ccaactgcta caagtgcgac     480

aagtgcaaga gcgagcagag caagaagaac aacaagaact ggatctggaa gaagagcagc     540

ggcaaggagg gcggcctgca gaaggagtac gccaacacca tcggcctgcc cccccgcacc      600

cagagcctgt gcctggtggt gtgcctggac gagaagggca agaagaccca ggagctgaag     660

aacatccgca ccaacagcga gctgctgaag gagtggatca tcgccgcctt ccacgagggc     720

aagaacctga agcccagcca cgagaagaag aacgacgaca acggcaagaa gctgtgcaag     780

gccctggagt acagcttcgc cgactacggc gacctgatca agggcaccag catctgggac     840

aacgagtaca ccaaggacct ggagctgaac ctgcagaaga tcttcggcaa gctgttccgc     900

aagtacatca agaagaacaa caccgccgag caggacacca gctacagcag cctggacgag     960

ctgcgcgaga gctggtggaa caccaacaag aagtacatct ggctggccat gaagcacggc    1020

gccggcatga acagcaccac ctgctgcggc gacggcagcg tgaccggcag cggcagcagc    1080

tgcgacgaca tccccaccat cgacctgatc ccccagtacc tgcgcttcct gcaggagtgg    1140

gtggagcact tctgcaagca gcgccaggag aaggtgaagc ccgtgatcga gaactgcaag    1200

agctgcaagg agagcggcgg cacctgcaac ggcgagtgca agaccgagtg caagaacaag    1260

tgcgaggtgt acaagaagtt catcgaggac tgcaagggcg gcgacggcac cgccggcagc    1320

agctgggtga agcgctggga ccagatctac aagcgctaca gcaagtacat cgaggacgcc    1380

aagcgcaacc gcaaggccgg caccaagaac tgcggcccca gcagcaccac caacgccgcc    1440

gagaacaagt gcgtgcagag cgacatcgac agcttcttca agcacctgat cgacatcggc    1500

ctgaccaccc ccagcagcta cctgagcatc gtgctggacg acaacatctg cggcgccgac    1560

aaggccccct ggaccaccta caccacctac accaccaccg agaagtgcaa caaggagacc 1620

gacaagagca agctgcagca gtgcaacacc gccgtggtgg tgaacgtgcc cagccccctg 1680

ggcaacaccc cccacggcta caagtacgcc tgccagtgca agatccccac caacgaggag 1740

acctgcgacg accgcaagga gtacatgaac cagtggagct gcggcagcgc ccgcaccatg 1800

aagcgcggct acaagaacga caactacgag ctgtgcaagt acaacggcgt ggacgtgaag 1860

cccaccaccg tgcgcagcaa cagcagcaag ctggacacca ccacccccgc cccccgcccc 1920

cccacccccg cccccaccat cgccagccag cccctgagcc tgcgccccga ggcctgccgc 1980

cccgccgccg gcggcgccgt gcacacccgc ggcctggact tcgcctgcga cttctgggtg 2040

ctggtggtgg tgggcggcgt gctggcctgc tacagcctgc tggtgaccgt ggccttcatc 2100

atcttctggg tgcgcagcaa gcgcagccgc ctgctgcaca gcgactacat gaacatgacc 2160

ccccgccgcc ccggccccac ccgcaagcac taccagccct acgccccccc ccgcgacttc 2220

gccgcctacc gcagcaagcg cggccgcaag aagctgctgt acatcttcaa gcagcccttc 2280

atgcgccccg tgcagaccac ccaggaggag gacggctgca gctgccgctt ccccgaggag 2340

gaggagggcg gctgcgagct gcgcgtgaag ttcagccgca gcgccgacgc ccccgcctac 2400

cagcagggcc agaaccagct gtacaacgag ctgaacctgg gccgccgcga ggagtacgac 2460

gtgctggaca agcgccgcgg ccgcgacccc gagatgggcg gcaagcccca gcgccgcaag 2520

aacccccagg agggcctgta caacgagctg cagaaggaca agatggccga ggcctacagc 2580

gagatcggca tgaagggcga gcgccgccgc ggcaagggcc acgacggcct gtaccagggc 2640

ctgagcaccg ccaccaagga cacctacgac gccctgcaca tgcaggccct gcccccccgc 2700

<210> 3
<211> 2646
<212> PRT
<213> artificial sequence

<220>
<223> amino acid sequence of VAR2CSA protein of Plasmodium falciparum
strain pf 3D7

<400> 3
   Met Asp Lys Ser Ser Ile Ala Asn Lys Ile Glu Ala Tyr Leu Gly Ala
   1 5 10 15
   Lys Ser Asp Asp Ser Lys Ile Asp Gln Ser Leu Lys Ala Asp Pro Ser
   20 25 30
   Glu Val Gln Tyr Tyr Gly Ser Gly Gly Asp Gly Tyr Tyr Leu Arg Lys
   35 40 45
   Asn Ile Cys Lys Ile Thr Val Asn His Ser Asp Ser Gly Thr Asn Asp
   50                  55                  60
   Pro Cys Asp Arg Ile Pro Pro Pro Tyr Gly Asp Asn Asp Gln Trp Lys
   65                  70                  75                  80
   Cys Ala Ile Ile Leu Ser Lys Val Ser Glu Lys Pro Glu Asn Val Phe
                   85                  90                  95
   Val Pro Pro Arg Arg Gln Arg Met Cys Ile Asn Asn Leu Glu Lys Leu
               100                 105                 110
   Asn Val Asp Lys Ile Arg Asp Lys His Ala Phe Leu Ala Asp Val Leu
           115                 120                 125
   Leu Thr Ala Arg Asn Glu Gly Glu Arg Ile Val Gln Asn His Pro Asp
       130                 135                 140
   Thr Asn Ser Ser Asn Val Cys Asn Ala Leu Glu Arg Ser Phe Ala Asp
   145                 150                 155                 160
   Ile Ala Asp Ile Ile Arg Gly Thr Asp Leu Trp Lys Gly Thr Asn Ser
                   165                 170                 175
   Asn Leu Glu Gln Asn Leu Lys Gln Met Phe Ala Lys Ile Arg Glu Asn
               180                 185                 190
   Asp Lys Val Leu Gln Asp Lys Tyr Pro Lys Asp Gln Asn Tyr Arg Lys
           195                 200                 205
   Leu Arg Glu Asp Trp Trp Asn Ala Asn Arg Gln Lys Val Trp Glu Val
       210                 215                 220
   Ile Thr Cys Gly Ala Arg Ser Asn Asp Leu Leu Ile Lys Arg Gly Trp
   225                 230                 235                 240
   Arg Thr Ser Gly Lys Ser Asn Gly Asp Asn Lys Leu Glu Leu Cys Arg
                   245                 250                 255
   Lys Cys Gly His Tyr Glu Glu Lys Val Pro Thr Lys Leu Asp Tyr Val
               260                 265                 270
   Pro Gln Phe Leu Arg Trp Leu Thr Glu Trp Ile Glu Asp Phe Tyr Arg
           275                 280                 285
   Glu Lys Gln Asn Leu Ile Asp Asp Met Glu Arg His Arg Glu Glu Cys
       290                 295                 300
   Thr Ser Glu Asp His Lys Ser Lys Glu Gly Thr Ser Tyr Cys Ser Thr
   305                 310                 315                 320
   Cys Lys Asp Lys Cys Lys Lys Tyr Cys Glu Cys Val Lys Lys Trp Lys
                   325                 330                 335
   Ser Glu Trp Glu Asn Gln Lys Asn Lys Tyr Thr Glu Leu Tyr Gln Gln
               340                 345                 350
   Asn Lys Asn Glu Thr Ser Gln Lys Asn Thr Ser Arg Tyr Asp Asp Tyr
           355                 360                 365
   Val Lys Asp Phe Phe Lys Lys Leu Glu Ala Asn Tyr Ser Ser Leu Glu
       370                 375                 380
   Asn Tyr Ile Lys Gly Asp Pro Tyr Phe Ala Glu Tyr Ala Thr Lys Leu
   385                 390                 395                 400
   Ser Phe Ile Leu Asn Ser Ser Asp Ala Asn Asn Pro Ser Glu Lys Ile
                   405                 410                 415
   Gln Lys Asn Asn Asp Glu Val Cys Asn Cys Asn Glu Ser Gly Ile Ala
               420                 425                 430
   Ser Val Glu Gln Glu Gln Ile Ser Asp Pro Ser Ser Asn Lys Thr Cys
           435                 440                 445
   Ile Thr His Ser Ser Ile Lys Ala Asn Lys Lys Lys Val Cys Lys His
       450                 455                 460
   Val Lys Leu Gly Val Arg Glu Asn Asp Lys Asp Leu Arg Val Cys Val
   465                 470                 475                 480
   Ile Glu His Thr Ser Leu Ser Gly Val Glu Asn Cys Cys Cys Gln Asp
                   485                 490                 495
   Phe Leu Arg Ile Leu Gln Glu Asn Cys Ser Asp Asn Lys Ser Gly Ser
               500                 505                 510
   Ser Ser Asn Gly Ser Cys Asn Asn Lys Asn Gln Glu Ala Cys Glu Lys
           515                 520                 525
   Asn Leu Glu Lys Val Leu Ala Ser Leu Thr Asn Cys Tyr Lys Cys Asp
       530                 535                 540
   Lys Cys Lys Ser Glu Gln Ser Lys Lys Asn Asn Lys Asn Trp Ile Trp
   545                 550                 555                 560
   Lys Lys Ser Ser Gly Lys Glu Gly Gly Leu Gln Lys Glu Tyr Ala Asn
                   565                 570                 575
   Thr Ile Gly Leu Pro Pro Arg Thr Gln Ser Leu Cys Leu Val Val Cys
               580                 585                 590
   Leu Asp Glu Lys Gly Lys Lys Thr Gln Glu Leu Lys Asn Ile Arg Thr
           595                 600                 605
   Asn Ser Glu Leu Leu Lys Glu Trp Ile Ile Ala Ala Phe His Glu Gly
       610                 615                 620
   Lys Asn Leu Lys Pro Ser His Glu Lys Lys Asn Asp Asp Asn Gly Lys
   625                 630                 635                 640
   Lys Leu Cys Lys Ala Leu Glu Tyr Ser Phe Ala Asp Tyr Gly Asp Leu
                   645                 650                 655
   Ile Lys Gly Thr Ser Ile Trp Asp Asn Glu Tyr Thr Lys Asp Leu Glu
               660                 665                 670
   Leu Asn Leu Gln Lys Ile Phe Gly Lys Leu Phe Arg Lys Tyr Ile Lys
           675                 680                 685
   Lys Asn Asn Thr Ala Glu Gln Asp Thr Ser Tyr Ser Ser Leu Asp Glu
       690                 695                 700
   Leu Arg Glu Ser Trp Trp Asn Thr Asn Lys Lys Tyr Ile Trp Leu Ala
   705                 710                 715                 720
   Met Lys His Gly Ala Gly Met Asn Ser Thr Thr Cys Cys Gly Asp Gly
                   725                 730                 735
   Ser Val Thr Gly Ser Gly Ser Ser Cys Asp Asp Ile Pro Thr Ile Asp
               740                 745                 750
   Leu Ile Pro Gln Tyr Leu Arg Phe Leu Gln Glu Trp Val Glu His Phe
           755                 760                 765
   Cys Lys Gln Arg Gln Glu Lys Val Lys Pro Val Ile Glu Asn Cys Lys
       770                 775                 780
   Ser Cys Lys Glu Ser Gly Gly Thr Cys Asn Gly Glu Cys Lys Thr Glu
   785                 790                 795                 800
   Cys Lys Asn Lys Cys Glu Val Tyr Lys Lys Phe Ile Glu Asp Cys Lys
                   805                 810                 815
   Gly Gly Asp Gly Thr Ala Gly Ser Ser Trp Val Lys Arg Trp Asp Gln
               820                 825                 830
   Ile Tyr Lys Arg Tyr Ser Lys Tyr Ile Glu Asp Ala Lys Arg Asn Arg
           835                 840                 845
   Lys Ala Gly Thr Lys Asn Cys Gly Pro Ser Ser Thr Thr Asn Ala Ala
       850                 855                 860
   Glu Asn Lys Cys Val Gln Ser Asp Ile Asp Ser Phe Phe Lys His Leu
   865                 870                 875                 880
   Ile Asp Ile Gly Leu Thr Thr Pro Ser Ser Tyr Leu Ser Ile Val Leu
                   885                 890                 895
   Asp Asp Asn Ile Cys Gly Ala Asp Lys Ala Pro Trp Thr Thr Tyr Thr
               900                 905                 910
   Thr Tyr Thr Thr Thr Glu Lys Cys Asn Lys Glu Thr Asp Lys Ser Lys
           915                 920                 925
   Leu Gln Gln Cys Asn Thr Ala Val Val Val Asn Val Pro Ser Pro Leu
       930                 935                 940
   Gly Asn Thr Pro His Gly Tyr Lys Tyr Ala Cys Gln Cys Lys Ile Pro
   945                 950                 955                 960
   Thr Asn Glu Glu Thr Cys Asp Asp Arg Lys Glu Tyr Met Asn Gln Trp
                   965                 970                 975
   Ser Cys Gly Ser Ala Arg Thr Met Lys Arg Gly Tyr Lys Asn Asp Asn
               980                 985                 990
   Tyr Glu Leu Cys Lys Tyr Asn Gly  Val Asp Val Lys Pro  Thr Thr Val
           995                 1000                1005
   Arg Ser  Asn Ser Ser Lys Leu  Asp Asp Lys Asp Val  Thr Phe Phe
       1010                1015                1020
   Asn Leu  Phe Glu Gln Trp Asn  Lys Glu Ile Gln Tyr  Gln Ile Glu
       1025                1030                1035
   Gln Tyr  Met Thr Asn Thr Lys  Ile Ser Cys Asn Asn  Glu Lys Asn
       1040                1045                1050
   Val Leu  Ser Arg Val Ser Asp  Glu Ala Ala Gln Pro  Lys Phe Ser
   1055                1060                1065
   Asp Asn Glu Arg Asp Arg Asn Ser Ile Thr His Glu Asp Lys Asn
       1070                1075                1080
   Cys Lys Glu Lys Cys Lys Cys Tyr Ser Leu Trp Ile Glu Lys Ile
       1085                1090                1095
   Asn Asp Gln Trp Asp Lys Gln Lys Asp Asn Tyr Asn Lys Phe Gln
       1100                1105                1110
   Arg Lys Gln Ile Tyr Asp Ala Asn Lys Gly Ser Gln Asn Lys Lys
       1115                1120                1125
   Val Val Ser Leu Ser Asn Phe Leu Phe Phe Ser Cys Trp Glu Glu
       1130                1135                1140
   Tyr Ile Gln Lys Tyr Phe Asn Gly Asp Trp Ser Lys Ile Lys Asn
       1145                1150                1155
   Ile Gly Ser Asp Thr Phe Glu Phe Leu Ile Lys Lys Cys Gly Asn
       1160                1165                1170
   Asp Ser Gly Asp Gly Glu Thr Ile Phe Ser Glu Lys Leu Asn Asn
       1175                1180                1185
   Ala Glu Lys Lys Cys Lys Glu Asn Glu Ser Thr Asn Asn Lys Met
       1190                1195                1200
   Lys Ser Ser Glu Thr Ser Cys Asp Cys Ser Glu Pro Ile Tyr Ile
       1205                1210                1215
   Arg Gly Cys Gln Pro Lys Ile Tyr Asp Gly Lys Ile Phe Pro Gly
       1220                1225                1230
   Lys Gly Gly Glu Lys Gln Trp Ile Cys Lys Asp Thr Ile Ile His
       1235                1240                1245
   Gly Asp Thr Asn Gly Ala Cys Ile Pro Pro Arg Thr Gln Asn Leu
       1250                1255                1260
   Cys Val Gly Glu Leu Trp Asp Lys Arg Tyr Gly Gly Arg Ser Asn
       1265                1270                1275
   Ile Lys Asn Asp Thr Lys Glu Ser Leu Lys Gln Lys Ile Lys Asn
       1280                1285                1290
   Ala Ile Gln Lys Glu Thr Glu Leu Leu Tyr Glu Tyr His Asp Lys
   1295 1300 1305
   Gly Thr Ala Ile Ile Ser Arg Asn Pro Met Lys Gly Gln Lys Glu
   1310 1315 1320
   Lys Glu Glu Lys Asn Asn Asp Ser Asn Gly Leu Pro Lys Gly Phe
   1325 1330 1335
   Cys His Ala Val Gln Arg Ser Phe Ile Asp Tyr Lys Asn Met Ile
   1340 1345 1350
   Leu Gly Thr Ser Val Asn Ile Tyr Glu Tyr Ile Gly Lys Leu Gln
   1355 1360 1365
   Glu Asp Ile Lys Lys Ile Ile Glu Lys Gly Thr Thr Lys Gln Asn
   1370 1375 1380
   Gly Lys Thr Val Gly Ser Gly Ala Glu Asn Val Asn Ala Trp Trp
   1385 1390 1395
   Lys Gly Ile Glu Gly Glu Met Trp Asp Ala Val Arg Cys Ala Ile
   1400 1405 1410
   Thr Lys Ile Asn Lys Lys Gln Lys Lys Asn Gly Thr Phe Ser Ile
   1415 1420 1425
   Asp Glu Cys Gly Ile Phe Pro Pro Thr Gly Asn Asp Glu Asp Gln
   1430 1435 1440
   Ser Val Ser Trp Phe Lys Glu Trp Ser Glu Gln Phe Cys Ile Glu
   1445 1450 1455
   Arg Leu Gln Tyr Glu Lys Asn Ile Arg Asp Ala Cys Thr Asn Asn
   1460 1465 1470
   Gly Gln Gly Asp Lys Ile Gln Gly Asp Cys Lys Arg Lys Cys Glu
   1475 1480 1485
   Glu Tyr Lys Lys Tyr Ile Ser Glu Lys Lys Gln Glu Trp Asp Lys
   1490 1495 1500
   Gln Lys Thr Lys Tyr Glu Asn Lys Tyr Val Gly Lys Ser Ala Ser
   1505 1510 1515
   Asp Leu Leu Lys Glu Asn Tyr Pro Glu Cys Ile Ser Ala Asn Phe
   1520 1525 1530
   Asp Phe Ile Phe Asn Asp Asn Ile Glu Tyr Lys Thr Tyr Tyr Pro
   1535 1540 1545
   Tyr Gly Asp Tyr Ser Ser Ile Cys Ser Cys Glu Gln Val Lys Tyr
   1550 1555 1560
   Tyr Glu Tyr Asn Asn Ala Glu Lys Lys Asn Asn Lys Ser Leu Cys
   1565 1570 1575
   His Glu Lys Gly Asn Asp Arg Thr Trp Ser Lys Lys Tyr Ile Lys
   1580 1585 1590
   Lys Leu Glu Asn Gly Arg Thr Leu Glu Gly Val Tyr Val Pro Pro
   1595 1600 1605
   Arg Arg Gln Gln Leu Cys Leu Tyr Glu Leu Phe Pro Ile Ile Ile
   1610 1615 1620
   Lys Asn Lys Asn Asp Ile Thr Asn Ala Lys Lys Glu Leu Leu Glu
   1625 1630 1635
   Thr Leu Gln Ile Val Ala Glu Arg Glu Ala Tyr Tyr Leu Trp Lys
   1640 1645 1650
   Gln Tyr His Ala His Asn Asp Thr Thr Tyr Leu Ala His Lys Lys
   1655 1660 1665
   Ala Cys Cys Ala Ile Arg Gly Ser Phe Tyr Asp Leu Glu Asp Ile
   1670 1675 1680
   Ile Lys Gly Asn Asp Leu Val His Asp Glu Tyr Thr Lys Tyr Ile
   1685 1690 1695
   Asp Ser Lys Leu Asn Glu Ile Phe Asp Ser Ser Asn Lys Asn Asp
   1700 1705 1710
   Ile Glu Thr Lys Arg Ala Arg Thr Asp Trp Trp Glu Asn Glu Ala
   1715 1720 1725
   Ile Ala Val Pro Asn Ile Thr Gly Ala Asn Lys Ser Asp Pro Lys
   1730 1735 1740
   Thr Ile Arg Gln Leu Val Trp Asp Ala Met Gln Ser Gly Val Arg
   1745 1750 1755
   Lys Ala Ile Asp Glu Glu Lys Glu Lys Lys Lys Pro Asn Glu Asn
   1760 1765 1770
   Phe Pro Pro Cys Met Gly Val Gln His Ile Gly Ile Ala Lys Pro
   1775 1780 1785
   Gln Phe Ile Arg Trp Leu Glu Glu Trp Thr Asn Glu Phe Cys Glu
   1790 1795 1800
   Lys Tyr Thr Lys Tyr Phe Glu Asp Met Lys Ser Asn Cys Asn Leu
   1805 1810 1815
   Arg Lys Gly Ala Asp Asp Cys Asp Asp Asn Ser Asn Ile Glu Cys
   1820 1825 1830
   Lys Lys Ala Cys Ala Asn Tyr Thr Asn Trp Leu Asn Pro Lys Arg
   1835 1840 1845
   Ile Glu Trp Asn Gly Met Ser Asn Tyr Tyr Asn Lys Ile Tyr Arg
   1850 1855 1860
   Lys Ser Asn Lys Glu Ser Glu Asp Gly Lys Asp Tyr Ser Met Ile
   1865 1870 1875
   Met Glu Pro Thr Val Ile Asp Tyr Leu Asn Lys Arg Cys Asn Gly
   1880 1885 1890
   Glu Ile Asn Gly Asn Tyr Ile Cys Cys Ser Cys Lys Asn Ile Gly
   1895 1900 1905
   Glu Asn Ser Thr Ser Gly Thr Val Asn Lys Lys Leu Gln Lys Lys
   1910 1915 1920
   Glu Thr Gln Cys Glu Asp Asn Lys Gly Pro Leu Asp Leu Met Asn
   1925 1930 1935
   Lys Val Leu Asn Lys Met Asp Pro Lys Tyr Ser Glu His Lys Met
   1940 1945 1950
   Lys Cys Thr Glu Val Tyr Leu Glu His Val Glu Glu Gln Leu Lys
   1955 1960 1965
   Glu Ile Asp Asn Ala Ile Lys Asp Tyr Lys Leu Tyr Pro Leu Asp
   1970 1975 1980
   Arg Cys Phe Asp Asp Lys Ser Lys Met Lys Val Cys Asp Leu Ile
   1985 1990 1995
   Gly Asp Ala Ile Gly Cys Lys His Lys Thr Lys Leu Asp Glu Leu
   2000                2005                2010
   Asp Glu Trp Asn Asp Val Asp Met Arg Asp Pro Tyr Asn Lys Tyr
       2015                2020                2025
   Lys Gly Val Leu Ile Pro Pro Arg Arg Arg Gln Leu Cys Phe Ser
       2030                2035                2040
   Arg Ile Val Arg Gly Pro Ala Asn Leu Arg Asn Leu Lys Glu Phe
       2045                2050                2055
   Lys Glu Glu Ile Leu Lys Gly Ala Gln Ser Glu Gly Lys Phe Leu
       2060                2065                2070
   Gly Asn Tyr Tyr Asn Glu Asp Lys Asp Lys Glu Lys Ala Leu Glu
       2075                2080                2085
   Ala Met Lys Asn Ser Phe Tyr Asp Tyr Glu Tyr Ile Ile Lys Gly
       2090                2095                2100
   Ser Asp Met Leu Thr Asn Ile Gln Phe Lys Asp Ile Lys Arg Lys
       2105                2110                2115
   Leu Asp Arg Leu Leu Glu Lys Glu Thr Asn Asn Thr Glu Lys Val
       2120                2125                2130
   Asp Asp Trp Trp Glu Thr Asn Lys Lys Ser Ile Trp Asn Ala Met
       2135                2140                2145
   Leu Cys Gly Tyr Lys Lys Ser Gly Asn Lys Ile Ile Asp Pro Ser
       2150                2155                2160
   Trp Cys Thr Ile Pro Thr Thr Glu Thr Pro Pro Gln Phe Leu Arg
       2165                2170                2175
   Trp Ile Lys Glu Trp Gly Thr Asn Val Cys Ile Gln Lys Glu Glu
       2180                2185                2190
   His Lys Glu Tyr Val Lys Ser Lys Cys Ser Asn Val Thr Asn Leu
       2195                2200                2205
   Gly Ala Gln Glu Ser Glu Ser Lys Asn Cys Thr Ser Glu Ile Lys
       2210                2215                2220
   Lys Tyr Gln Glu Trp Ser Arg Lys Arg Ser Ile Gln Trp Glu Ala
       2225                2230                2235
   Ile Ser Glu Gly Tyr Lys Lys Tyr Lys Gly Met Asp Glu Phe Lys
       2240                2245                2250
   Asn Thr Phe Lys Asn Ile Lys Glu Pro Asp Ala Asn Glu Pro Asn
       2255                2260                2265
   Ala Asn Glu Tyr Leu Lys Lys His Cys Ser Lys Cys Pro Cys Gly
       2270                2275                2280
   Phe Asn Asp Met Gln Glu Ile Thr Lys Tyr Thr Asn Ile Gly Asn
       2285                2290                2295
   Glu Ala Phe Lys Gln Ile Lys Glu Gln Val Asp Ile Pro Ala Glu
       2300                2305                2310
   Leu Glu Asp Val Ile Tyr Arg Leu Lys His His Glu Tyr Asp Lys
       2315                2320                2325
   Gly Asn Asp Tyr Ile Cys Asn Lys Tyr Lys Asn Ile Asn Val Asn
       2330                2335                2340
   Met Lys Lys Asn Asn Asp Asp Thr Trp Thr Asp Leu Val Lys Asn
       2345                2350                2355
   Ser Ser Asp Ile Asn Lys Gly Val Leu Leu Pro Pro Arg Arg Lys
       2360                2365                2370
   Asn Leu Phe Leu Lys Ile Asp Glu Ser Asp Ile Cys Lys Tyr Lys
       2375                2380                2385
   Arg Asp Pro Lys Leu Phe Lys Asp Phe Ile Tyr Ser Ser Ala Ile
       2390                2395                2400
   Ser Glu Val Glu Arg Leu Lys Lys Val Tyr Gly Glu Ala Lys Thr
       2405                2410                2415
   Lys Val Val His Ala Met Lys Tyr Ser Phe Ala Asp Ile Gly Ser
       2420                2425                2430
   Ile Ile Lys Gly Asp Asp Met Met Glu Asn Asn Ser Ser Asp Lys
       2435                2440                2445
   Ile Gly Lys Ile Leu Gly Asp Gly Val Gly Gln Asn Glu Lys Arg
       2450                2455                2460
   Lys Lys Trp Trp Asp Met Asn Lys Tyr His Ile Trp Glu Ser Met
       2465                2470                2475
   Leu Cys Gly Tyr Lys His Ala Tyr Gly Asn Ile Ser Glu Asn Asp
   2480 2485 2490
   Arg Lys Met Leu Asp Ile Pro Asn Asn Asp Asp Glu His Gln Phe
   2495 2500 2505
   Leu Arg Trp Phe Gln Glu Trp Thr Glu Asn Phe Cys Thr Lys Arg
   2510 2515 2520
   Asn Glu Leu Tyr Glu Asn Met Val Thr Ala Cys Asn Ser Ala Lys
   2525 2530 2535
   Cys Asn Thr Ser Asn Gly Ser Val Asp Lys Lys Glu Cys Thr Glu
   2540 2545 2550
   Ala Cys Lys Asn Tyr Ser Asn Phe Ile Leu Ile Lys Lys Lys Glu
   2555 2560 2565
   Tyr Gln Ser Leu Asn Ser Gln Tyr Asp Met Asn Tyr Lys Glu Thr
   2570 2575 2580
   Lys Ala Glu Lys Lys Glu Ser Pro Glu Tyr Phe Lys Asp Lys Cys
   2585 2590 2595
   Asn Gly Glu Cys Ser Cys Leu Ser Glu Tyr Phe Lys Asp Glu Thr
   2600 2605 2610
   Arg Trp Lys Asn Pro Tyr Glu Thr Leu Asp Asp Thr Glu Val Lys
   2615 2620 2625
   Asn Asn Cys Met Cys Lys Pro Pro Pro Pro Ala Ser Asn Ser Gly
   2630 2635 2640
   Arg Glu Leu
   2645

<210> 4
<211> 7938
<212> DNA
<213> artificial sequence

<220>
<223> nucleotide sequence encoding VAR2CSA protein of Plasmodium falciparum
strain pf 3D7

<400> 4
atggacaagt cctctatcgc caacaagatc gaagcctacc tgggagccaa gtctgacgac 60

tctaagatcg accagtctct gaaggccgac ccctctgaag tccagtacta cggatctggt 120

ggcgacggtt actacctgag gaagaacatc tgcaagatca ccgtcaacca ctctgactct 180

ggcaccaacg acccctgcga caggatcccc cctccctacg gcgacaacga ccagtggaag 240

tgcgccatca tcctgtctaa ggtgtccgaa aagcccgaaa acgtgttcgt cccccctagg 300

cgccagagga tgtgcatcaa caacctggaa aagctgaacg tcgacaagat cagggacaag 360

cacgccttcc tcgccgacgt cctgctgacc gctaggaacg aaggcgaaag gatcgtccag 420

aaccaccccg acaccaactc ttctaacgtc tgcaacgccc tggaacgctc tttcgccgac 480

atcgctgaca tcatccgcgg aaccgacctg tggaagggca ccaactctaa cctggaacag 540

aacctgaagc agatgttcgc caagatccgc gaaaacgaca aggtcctgca ggataagtac 600

cccaaggacc agaactaccg caagctgcgc gaggactggt ggaacgccaa caggcagaag 660

gtctgggaag tcatcacttg cggagccagg tctaacgacc tgctgatcaa gaggggatgg 720

cgcacctctg gaaagtctaa cggcgacaac aagctggaac tgtgccgcaa gtgcggacac 780

tacgaagaaa aggtccccac caagctggac tacgtccccc agttcctgcg ctggctgacc 840

gaatggatcg aggacttcta cagggaaaag cagaacctga tcgacgacat ggaaaggcac 900

agggaagaat gcacctctga ggaccacaag tctaaggaag gcacctctta ctgctctacc 960

tgcaaggaca agtgcaagaa gtactgcgag tgcgtcaaga agtggaagtc tgaatgggag 1020

aaccagaaga acaagtacac cgaactgtac cagcagaaca agaacgaaac ctctcagaag 1080

aacacctctc gctacgacga ctacgtcaag gacttcttca agaagctcga ggccaactac 1140

tcctccctgg aaaactacat caagggcgac ccctacttcg ccgaatacgc taccaagctg 1200

tccttcatcc tgaactcttc cgacgccaac aacccctctg agaagatcca aaagaacaac 1260

gacgaagtct gcaactgcaa cgaatctgga atcgcctctg tcgaacaaga gcagatctct 1320

gacccctcta gcaacaagac ctgcatcacc cactcttcta tcaaggccaa caagaagaag 1380

gtctgcaagc acgtcaagct gggagtcaga gagaacgaca aggacctccg cgtctgcgtc 1440

atcgaacaca cctccctgtc tggtgtcgaa aactgctgct gccaggactt cctgaggatc 1500

ctgcaagaaa actgctccga caacaagtcc ggatcttctt ctaacggctc ctgcaacaac 1560

aagaaccagg aagcctgcga gaagaacctc gaaaaggtcc tggcctctct gaccaactgc 1620

tacaagtgcg acaagtgcaa gtctgagcag agcaagaaga acaacaagaa ctggatctgg 1680

aagaagtcct ccggaaagga aggtggactg cagaaggaat acgccaacac catcggactg 1740

ccccccagga cccagtctct gtgcctggtc gtctgcctgg acgaaaaggg aaagaagacc 1800

caagagctga agaacatccg caccaactcc gaactgctga aggaatggat catcgccgcc 1860

ttccacgagg gaaagaacct caagccctct cacgaaaaga agaacgacga caacggcaag 1920

aagctgtgca aggccctcga gtactccttc gccgactacg gcgacctgat caaggaaacc 1980

tctatctggg acaacgagta caccaaggac ttggaactga acctgcaaaa gatcttcggc 2040

aagctgttcc gcaagtacat caagaagaac aacaccgccg agcaggacac ctcctactct 2100

agcctggacg agctgaggga atcttggtgg aacactaaca agaagtacat ctggctggcc 2160

atgaagcacg gtgccggaat gaactctacc acttgctgcg gcgacggatc tgtgaccgga 2220

tctggatctt cttgcgacga catccccacc atcgacctga tccctcagta cctgcgcttc 2280

ctgcaagagt gggtcgaaca cttctgcaag cagaggcaag agaaggtcaa gcccgtcatc 2340

gagaactgca agtcttgcaa ggaaagcgga ggaacctgca acggcgaatg caagaccgag 2400

tgcaagaaca agtgtgaagt ctacaagaag ttcatcgagg actgcaaggg tggcgacgga 2460

accgccggat cttcctgggt caagcgctgg gaccagatct acaagaggta ctctaagtac 2520

atcgaggacg ccaagaggaa ccgcaaggcc ggcaccaaga actgcggacc ctcttctacc 2580

accaacgccg ccgaaaacaa gtgcgtccag tctgacatcg actcattctt caagcacctc 2640

atcgacatcg gactgaccac cccctcctct tacctgtcta tcgtcctgga cgacaacatc 2700

tgcggagccg acaaggcccc ctggaccacc tacaccactt acaccaccac cgaaaagtgc 2760

aacaaggaaa ccgacaagtc caagctccag cagtgcaaca ccgctgtcgt cgtcaacgtc 2820

ccctctcccc tgggaaacac cccccacgga tacaagtacg cctgccagtg caagatcccc 2880

accaacgaag aaacctgcga cgaccgcaag gagtacatga accagtggtc ttgcggatct 2940

gccaggacca tgaagagggg atacaagaac gacaactacg aactgtgcaa gtacaacggt 3000

gtcgacgtca agcccaccac cgtcaggtct aactcctcta agctggacga caaggacgtc 3060

accttcttca acctgttcga gcagtggaac aaggaaatcc agtaccagat cgagcagtac 3120

atgaccaaca ccaagatcag ctgcaacaac gaaaagaacg tcctgtctcg cgtcagcgac 3180

gaagccgccc agcccaagtt ctctgacaac gaaagggacc gcaactctat cacccacgag 3240

gacaagaact gcaaggaaaa gtgcaagtgc tactccctgt ggatcgagaa gatcaacgac 3300

caatgggaca agcagaagga caactacaac aagttccagc gcaagcagat ctacgacgct 3360

aacaagggat cccaaaacaa gaaggtcgtc agcctgtcca acttcctgtt cttcagctgc 3420

tgggaagagt acatccagaa gtacttcaac ggcgactggt ccaagatcaa gaacatcgga 3480

tctgacacct tcgagttcct catcaagaag tgcggaaacg actctggcga cggcgaaacc 3540

atcttctctg agaagctgaa caacgccgag aagaagtgca aggaaaacga gtccaccaac 3600

aacaagatga agtcctctga aacctcctgc gactgctctg aacccatcta catcagggga 3660

tgccagccca agatctacga cggaaagatc ttccccggaa agggcggaga aaagcagtgg 3720

atctgcaagg acaccatcat ccacggcgac accaacggtg cctgcatccc tcctaggacc 3780

cagaacctgt gcgtcggaga actgtgggac aagcgctacg gtggaaggtc taacatcaag 3840

aacgacacca aggaatccct gaagcagaag atcaagaacg ccatccagaa ggaaaccgaa 3900

ctgctgtacg agtaccacga caagggaacc gccatcatct ctaggaaccc catgaaggga 3960

cagaaggaaa aggaagagaa gaacaacgac tccaacggac tgcccaaggg attctgccac 4020

gccgtccagc gcagcttcat cgactacaag aacatgatcc tgggcacctc cgtcaacatc 4080

tacgagtaca tcggaaagct gcaagaggac atcaagaaga tcatcgagaa gggaaccacc 4140

aagcagaacg gaaagaccgt cggaagcgga gccgaaaacg tcaacgcctg gtggaaggga 4200

atcgaaggcg agatgtggga cgccgtccgc tgcgccatca ctaagatcaa caagaagcag 4260

aagaagaacg gcaccttctc tatcgacgag tgcggaatct tcccccccac cggaaacgac 4320

gaggaccagt ctgtctcctg gttcaaggaa tggtccgaac agttctgcat cgaaaggctg 4380

cagtacgaga agaacatcag ggacgcctgc accaacaacg gacagggcga caagatccaa 4440

ggcgactgca agcgcaagtg cgaagagtac aagaagtaca tcagcgagaa gaagcaagag 4500

tgggacaagc aaaagactaa gtacgagaac aagtacgtcg gaaagtctgc ctctgacctg 4560

ctcaaggaaa actaccccga atgcatctct gccaacttcg acttcatctt caacgacaac 4620

atcgagtaca agacctacta cccttacggc gactactctt ccatctgctc ttgcgaacaa 4680

gtgaagtact acgagtacaa caacgctgaa aagaagaaca acaagtccct gtgccacgag 4740

aagggaaacg accgcacctg gtctaagaag tacatcaaga agctcgagaa cggaaggacc 4800

ctggaaggcg tctacgtccc accccgcagg cagcagctgt gcctgtacga actgttcccc 4860

atcatcatca agaacaagaa cgacatcacc aacgccaaga aggaattgct ggaaaccctg 4920

cagatcgtcg ccgaaaggga agcctactac ctctggaagc agtaccacgc ccacaacgac 4980

accacctacc tggcccacaa gaaggcctgc tgcgctatca ggggatcttt ctacgacctc 5040

gaggatatca tcaagggcaa cgacctggtc cacgacgagt acaccaagta catcgactct 5100

aagctgaacg agatcttcga ctcctccaac aagaacgaca tcgaaactaa gagggccagg 5160

accgactggt gggaaaacga agctatcgcc gtccccaaca tcaccggtgc caacaagtct 5220

gaccccaaga ccatcaggca gctcgtctgg gacgccatgc agtctggtgt ccgcaaggcc 5280

atcgacgaag agaaggaaaa gaagaagccc aacgaaaact tcccaccctg catgggagtc 5340

cagcacatcg gaatcgccaa gccccagttc atccgttggc tggaagaatg gaccaacgag 5400

ttctgcgaga agtacactaa gtacttcgag gacatgaagt ccaactgcaa cctgcgcaag 5460

ggcgccgacg actgcgacga caactctaac atcgaatgca agaaggcttg cgccaactac 5520

accaactggc tgaaccccaa gaggatcgaa tggaacggaa tgtccaacta ctacaacaag 5580

atctaccgca agtccaacaa ggaatctgag gacggaaagg actactcaat gatcatggaa 5640

cccaccgtga tcgactacct gaacaagagg tgtaacggcg agatcaacgg caactacatc 5700

tgctgctctt gcaagaacat cggcgagaac tctacctctg gcaccgtgaa caagaagctg 5760

caaaagaagg aaactcagtg cgaggacaac aagggacccc tggacctgat gaacaaggtc 5820

ctcaacaaga tggaccccaa gtactctgag cacaagatga agtgcaccga ggtctacctg 5880
gaacacgtcg aagaacagtt gaaggaaatc gacaacgcca tcaaggacta caagctgtac 6000
cctctggacc gctgcttcga cgacaagagc aagatgaagg tttgcgacct catcggcgac 6000
gccatcggat gcaagcacaa gaccaagctc gacgaactgg acgagtggaa cgacgtcgac 6060
atgagggacc cttacaacaa gtacaagggc gtcctgatcc ccccaaggcg caggcagctg 6120
tgcttctcta ggatcgtcag gggacccgcc aacctgagga acctgaagga gttcaaggaa 6180
gagatcctga agggcgccca gtctgaggga aagttcctgg gaaactacta caacgaggac 6240
aaggacaagg aaaaggccct ggaagccatg aagaacagct tctacgacta cgagtacatc 6300
atcaagggct ctgacatgct gaccaacatc cagttcaagg acatcaagcg caagctggac 6360
aggctgctgg aaaaggaaac taacaacacc gaaaaggtgg acgactggtg ggagactaac 6420
aagaagagta tctggaacgc catgttgtgc ggttacaaga agtccggcaa caagatcatc 6480
gacccctctt ggtgcaccat ccctaccacc gaaacccccc ctcagttctt gcgttggatc 6540
aaggaatggg gcaccaacgt ctgcatccaa aaggaagaac acaaggaata cgtcaagagc 6600
aagtgctcca acgtcaccaa cctcggagcc caagaatctg aatctaagaa ctgcacctcc 6660
gagatcaaga agtaccaaga gtggtcccgc aagcgctcta tccagtggga agccatctct 6720
gagggctaca agaagtacaa gggtatggac gagttcaaga acaccttcaa gaacatcaag 6780
gaacccgacg ccaacgagcc caacgccaac gaatacctga agaagcactg ctctaagtgc 6840
ccctgcggat tcaacgacat gcaagaaatc actaagtaca ccaacatcgg caacgaggcc 6900
ttcaagcaga tcaaggaaca ggtcgacatc cccgccgaat tggaggacgt catctacagg 6960
ctgaagcacc acgaatacga caagggaaac gactacatct gcaacaagta caagaacatc 7020
aacgtcaaca tgaagaagaa caacgacgac acctggaccg acctggtcaa gaactcttct 7080
gacatcaaca agggtgtcct gctgccccct cgccgcaaga acctgttcct gaagatcgac 7140
gaatccgaca tctgcaagta caagagggac cctaagctgt tcaaggactt catctactct 7200
tccgccatct ccgaagtcga acgcctcaag aaggtctacg gcgaggccaa gaccaaggtc 7260
gtccacgcta tgaagtactc attcgcagac atcggctcca tcatcaaggg cgacgacatg 7320
atggaaaaca acagctctga caagatcggc aagatcctgg gcgacggtgt cggccagaac 7380
gaaaagcgca agaagtggtg ggacatgaac aagtaccaca tctgggagtc tatgctgtgc 7440
ggctacaagc acgcttacgg aaacatctct gagaacgacc gaaagatgct ggacatcccc 7500
aacaacgacg acgaacacca gttcctccgc tggttccaag aatggactga aaacttctgc 7560
accaagcgca acgagctgta cgaaaacatg gtcaccgcct gcaactctgc caagtgcaac 7620
acttctaacg gatccgtgga caagaaggaa tgcaccgaag cctgcaagaa ctactccaac 7680

ttcatcctga tcaagaagaa ggaataccag tccctgaact cccagtacga catgaactac 7740

aaggaaacta aggccgaaaa gaaggaatcc cccgagtact tcaaggacaa gtgtaacggc 7800

gaatgctctt gcctgagcga atacttcaag gacgaaacca ggtggaagaa cccctacgaa 7860

accctcgacg acaccgaagt caagaacaac tgcatgtgca agcccccacc ccctgcctct 7920

aacagcggcc gcgagctc 7938

<210> 5
<211> 2649
<212> PRT
<213> artificial sequence

<220>
<223> amino acid sequence of VAR2CSA protein of Plasmodium falciparum
strain pf FCR3

<400> 5

Met Asp Ser Thr Ser Thr Ile Ala Asn Lys Ile Glu Glu Tyr Leu Gly
1               5                   10                  15

Ala Lys Ser Asp Asp Ser Lys Ile Asp Glu Leu Leu Lys Ala Asp Pro
            20                  25                  30

Ser Glu Val Glu Tyr Tyr Arg Ser Gly Gly Asp Gly Asp Tyr Leu Lys
        35                  40                  45

Asn Asn Ile Cys Lys Ile Thr Val Asn His Ser Asp Ser Gly Lys Tyr
    50                  55                  60

Asp Pro Cys Glu Lys Lys Leu Pro Pro Tyr Asp Asp Asn Asp Gln Trp
65                  70                  75                  80

Lys Cys Gln Gln Asn Ser Ser Asp Gly Ser Gly Lys Pro Glu Asn Ile
                85                  90                  95

Cys Val Pro Pro Arg Arg Glu Arg Leu Cys Thr Tyr Asn Leu Glu Asn
            100                 105                 110

Leu Lys Phe Asp Lys Ile Arg Asp Asn Asn Ala Phe Leu Ala Asp Val
        115                 120                 125

Leu Leu Thr Ala Arg Asn Glu Gly Glu Lys Ile Val Gln Asn His Pro
    130                 135                 140

Asp Thr Asn Ser Ser Asn Val Cys Asn Ala Leu Glu Arg Ser Phe Ala
145                 150                 155                 160

Asp Leu Ala Asp Ile Ile Arg Gly Thr Asp Gln Trp Lys Gly Thr Asn

                165                 170                 175

Ser Asn Leu Glu Lys Asn Leu Lys Gln Met Phe Ala Lys Ile Arg Glu
            180                 185                 190

Asn Asp Lys Val Leu Gln Asp Lys Tyr Pro Lys Asp Gln Lys Tyr Thr
        195                 200                 205

Lys Leu Arg Glu Ala Trp Trp Asn Ala Asn Arg Gln Lys Val Trp Glu
    210                 215                 220

Val Ile Thr Cys Gly Ala Arg Ser Asn Asp Leu Leu Ile Lys Arg Gly
225                 230                 235                 240

Trp Arg Thr Ser Gly Lys Ser Asp Arg Lys Lys Asn Phe Glu Leu Cys
                245                 250                 255

Arg Lys Cys Gly His Tyr Glu Lys Glu Val Pro Thr Lys Leu Asp Tyr
            260                 265                 270

Val Pro Gln Phe Leu Arg Trp Leu Thr Glu Trp Ile Glu Asp Phe Tyr
        275                 280                 285

Arg Glu Lys Gln Asn Leu Ile Asp Asp Met Glu Arg His Arg Glu Glu
    290                 295                 300

Cys Thr Arg Glu Asp His Lys Ser Lys Glu Gly Thr Ser Tyr Cys Ser
305                 310                 315                 320

Thr Cys Lys Asp Lys Cys Lys Lys Tyr Cys Glu Cys Val Lys Lys Trp
                325                 330                 335

Lys Thr Glu Trp Glu Asn Gln Glu Asn Lys Tyr Lys Asp Leu Tyr Glu
            340                 345                 350

Gln Asn Lys Asn Lys Thr Ser Gln Lys Asn Thr Ser Arg Tyr Asp Asp
        355                 360                 365

Tyr Val Lys Asp Phe Phe Glu Lys Leu Glu Ala Asn Tyr Ser Ser Leu
    370                 375                 380

Glu Asn Tyr Ile Lys Gly Asp Pro Tyr Phe Ala Glu Tyr Ala Thr Lys
385                 390                 395                 400

Leu Ser Phe Ile Leu Asn Pro Ser Asp Ala Asn Asn Pro Ser Gly Glu
                405                 410                 415

Thr Ala Asn His Asn Asp Glu Ala Cys Asn Cys Asn Glu Ser Gly Ile
            420                 425                 430

Ser Ser Val Gly Gln Ala Gln Thr Ser Gly Pro Ser Ser Asn Lys Thr
        435                 440                 445

Cys Ile Thr His Ser Ser Ile Lys Thr Asn Lys Lys Lys Glu Cys Lys
    450                 455                 460

Asp Val Lys Leu Gly Val Arg Glu Asn Asp Lys Asp Leu Lys Ile Cys
465                 470                 475                 480

Val Ile Glu Asp Thr Ser Leu Ser Gly Val Asp Asn Cys Cys Cys Gln
                485                 490                 495

Asp Leu Leu Gly Ile Leu Gln Glu Asn Cys Ser Asp Asn Lys Arg Gly
            500                 505                 510

Ser Ser Ser Asn Asp Ser Cys Asp Asn Lys Asn Gln Asp Glu Cys Gln
        515                 520                 525

Lys Lys Leu Glu Lys Val Phe Ala Ser Leu Thr Asn Gly Tyr Lys Cys
    530                 535                 540

Asp Lys Cys Lys Ser Gly Thr Ser Arg Ser Lys Lys Lys Trp Ile Trp
545                 550                 555                 560

Lys Lys Ser Ser Gly Asn Glu Glu Gly Leu Gln Glu Glu Tyr Ala Asn
                565                 570                 575

Thr Ile Gly Leu Pro Pro Arg Thr Gln Ser Leu Tyr Leu Gly Asn Leu
            580                 585                 590

Pro Lys Leu Glu Asn Val Cys Glu Asp Val Lys Asp Ile Asn Phe Asp
        595                 600                 605

Thr Lys Glu Lys Phe Leu Ala Gly Cys Leu Ile Val Ser Phe His Glu
    610                 615                 620

Gly Lys Asn Leu Lys Lys Arg Tyr Pro Gln Asn Lys Asn Ser Gly Asn
625                 630                 635                 640

Lys Glu Asn Leu Cys Lys Ala Leu Glu Tyr Ser Phe Ala Asp Tyr Gly
                645                 650                 655

Asp Leu Ile Lys Gly Thr Ser Ile Trp Asp Asn Glu Tyr Thr Lys Asp
            660                 665                 670

Leu Glu Leu Asn Leu Gln Asn Asn Phe Gly Lys Leu Phe Gly Lys Tyr
        675                 680                 685

Ile Lys Lys Asn Asn Thr Ala Glu Gln Asp Thr Ser Tyr Ser Ser Leu
    690                 695                 700

Asp Glu Leu Arg Glu Ser Trp Trp Asn Thr Asn Lys Lys Tyr Ile Trp
705                 710                 715                 720

Thr Ala Met Lys His Gly Ala Glu Met Asn Ile Thr Thr Cys Asn Ala
                725                 730                 735

Asp Gly Ser Val Thr Gly Ser Gly Ser Ser Cys Asp Asp Ile Pro Thr
            740                 745                 750

Ile Asp Leu Ile Pro Gln Tyr Leu Arg Phe Leu Gln Glu Trp Val Glu
        755                 760                 765

Asn Phe Cys Glu Gln Arg Gln Ala Lys Val Lys Asp Val Ile Thr Asn
    770                 775                 780

Cys Lys Ser Cys Lys Glu Ser Gly Asn Lys Cys Lys Thr Glu Cys Lys
785                 790                 795                 800

Thr Lys Cys Lys Asp Glu Cys Glu Lys Tyr Lys Lys Phe Ile Glu Ala
                805                 810                 815

Cys Gly Thr Ala Gly Gly Gly Ile Gly Thr Ala Gly Ser Pro Trp Ser
            820                 825                 830

Lys Arg Trp Asp Gln Ile Tyr Lys Arg Tyr Ser Lys His Ile Glu Asp
        835                 840                 845

Ala Lys Arg Asn Arg Lys Ala Gly Thr Lys Asn Cys Gly Thr Ser Ser
    850                 855                 860

Thr Thr Asn Ala Ala Ala Ser Thr Asp Glu Asn Lys Cys Val Gln Ser
865                 870                 875                 880

Asp Ile Asp Ser Phe Phe Lys His Leu Ile Asp Ile Gly Leu Thr Thr
                885                 890                 895

Pro Ser Ser Tyr Leu Ser Asn Val Leu Asp Asp Asn Ile Cys Gly Ala
            900                 905                 910

Asp Lys Ala Pro Trp Thr Thr Tyr Thr Thr Tyr Thr Thr Thr Glu Lys
        915                 920                 925

Cys Asn Lys Glu Arg Asp Lys Ser Lys Ser Gln Ser Ser Asp Thr Leu
    930                 935                 940

Val Val Val Asn Val Pro Ser Pro Leu Gly Asn Thr Pro Tyr Arg Tyr
945                 950                 955                 960

Lys Tyr Ala Cys Gln Cys Lys Ile Pro Thr Asn Glu Glu Thr Cys Asp
                965                 970                 975

Asp Arg Lys Glu Tyr Met Asn Gln Trp Ser Cys Gly Ser Ala Arg Thr
            980                 985                 990

Met Lys Arg Gly Tyr Lys Asn Asp  Asn Tyr Glu Leu Cys  Lys Tyr Asn
        995                 1000                1005

Gly Val  Asp Val Lys Pro Thr  Thr Val Arg Ser Asn  Ser Ser Lys
    1010                 1015                 1020

Leu Asp  Gly Asn Asp Val Thr  Phe Phe Asn Leu Phe  Glu Gln Trp
    1025                 1030                 1035

Asn Lys  Glu Ile Gln Tyr Gln  Ile Glu Gln Tyr Met  Thr Asn Ala
    1040                 1045                 1050

Asn Ile  Ser Cys Ile Asp Glu  Lys Glu Val Leu Asp  Ser Val Ser
    1055                 1060                 1065

Asp Glu  Gly Thr Pro Lys Val  Arg Gly Gly Tyr Glu  Asp Gly Arg
    1070                 1075                 1080

Asn Asn  Asn Thr Asp Gln Gly  Thr Asn Cys Lys Glu  Lys Cys Lys
    1085                 1090                 1095

Cys Tyr  Lys Leu Trp Ile Glu  Lys Ile Asn Asp Gln  Trp Gly Lys
    1100                 1105                 1110

Gln Lys  Asp Asn Tyr Asn Lys  Phe Arg Ser Lys Gln  Ile Tyr Asp
    1115                 1120                 1125

Ala Asn  Lys Gly Ser Gln Asn  Lys Lys Val Val Ser  Leu Ser Asn
    1130                 1135                 1140

Phe Leu  Phe Phe Ser Cys Trp  Glu Glu Tyr Ile Gln  Lys Tyr Phe
    1145                 1150                 1155

Asn Gly  Asp Trp Ser Lys Ile  Lys Asn Ile Gly Ser  Asp Thr Phe

1160                1165                1170

Glu Phe Leu Ile Lys Lys Cys Gly Asn Asn Ser Ala His Gly Glu
    1175                1180                1185

Glu Ile Phe Ser Glu Lys Leu Lys Asn Ala Glu Lys Lys Cys Lys
    1190                1195                1200

Glu Asn Glu Ser Thr Asp Thr Asn Ile Asn Lys Ser Glu Thr Ser
    1205                1210                1215

Cys Asp Leu Asn Ala Thr Asn Tyr Ile Arg Gly Cys Gln Ser Lys
    1220                1225                1230

Thr Tyr Asp Gly Lys Ile Phe Pro Gly Lys Gly Gly Glu Lys Gln
    1235                1240                1245

Trp Ile Cys Lys Asp Thr Ile Ile His Gly Asp Thr Asn Gly Ala
    1250                1255                1260

Cys Ile Pro Pro Arg Thr Gln Asn Leu Cys Val Gly Glu Leu Trp
    1265                1270                1275

Asp Lys Ser Tyr Gly Gly Arg Ser Asn Ile Lys Asn Asp Thr Lys
    1280                1285                1290

Glu Leu Leu Lys Glu Lys Ile Lys Asn Ala Ile His Lys Glu Thr
    1295                1300                1305

Glu Leu Leu Tyr Glu Tyr His Asp Thr Gly Thr Ala Ile Ile Ser
    1310                1315                1320

Lys Asn Asp Lys Lys Gly Gln Lys Gly Lys Asn Asp Pro Asn Gly
    1325                1330                1335

Leu Pro Lys Gly Phe Cys His Ala Val Gln Arg Ser Phe Ile Asp
    1340                1345                1350

Tyr Lys Asn Met Ile Leu Gly Thr Ser Val Asn Ile Tyr Glu His
    1355                1360                1365

Ile Gly Lys Leu Gln Glu Asp Ile Lys Lys Ile Ile Glu Lys Gly
    1370                1375                1380

Thr Pro Gln Gln Lys Asp Lys Ile Gly Gly Val Gly Ser Ser Thr
    1385                1390                1395

Glu Asn Val Asn Ala Trp Trp Lys Gly Ile Glu Arg Glu Met Trp
    1400                1405                1410

Asp Ala Val Arg Cys Ala Ile Thr Lys Ile Asn Lys Lys Asn Asn
    1415                1420                1425

Asn Ser Ile Phe Asn Gly Asp Glu Cys Gly Val Ser Pro Pro Thr
    1430                1435                1440

Gly Asn Asp Glu Asp Gln Ser Val Ser Trp Phe Lys Glu Trp Gly
    1445                1450                1455

Glu Gln Phe Cys Ile Glu Arg Leu Arg Tyr Glu Gln Asn Ile Arg
    1460                1465                1470

Glu Ala Cys Thr Ile Asn Gly Lys Asn Glu Lys Lys Cys Ile Asn
    1475                1480                1485

Ser Lys Ser Gly Gln Gly Asp Lys Ile Gln Gly Ala Cys Lys Arg
    1490                1495                1500

Lys Cys Glu Lys Tyr Lys Lys Tyr Ile Ser Glu Lys Lys Gln Glu
    1505                1510                1515

Trp Asp Lys Gln Lys Thr Lys Tyr Glu Asn Lys Tyr Val Gly Lys
    1520                1525                1530

Ser Ala Ser Asp Leu Leu Lys Glu Asn Tyr Pro Glu Cys Ile Ser
    1535                1540                1545

Ala Asn Phe Asp Phe Ile Phe Asn Asp Asn Ile Glu Tyr Lys Thr
    1550                1555                1560

Tyr Tyr Pro Tyr Gly Asp Tyr Ser Ser Ile Cys Ser Cys Glu Gln
    1565                1570                1575

Val Lys Tyr Tyr Lys Tyr Asn Asn Ala Glu Lys Lys Asn Asn Lys
    1580                1585                1590

Ser Leu Cys Tyr Glu Lys Asp Asn Asp Met Thr Trp Ser Lys Lys
    1595                1600                1605

Tyr Ile Lys Lys Leu Glu Asn Gly Arg Ser Leu Glu Gly Val Tyr
    1610                1615                1620

Val Pro Pro Arg Arg Gln Gln Leu Cys Leu Tyr Glu Leu Phe Pro
    1625                1630                1635

Ile Ile Ile Lys Asn Glu Glu Gly Met Glu Lys Ala Lys Glu Glu
    1640                1645                1650

Leu Leu Glu Thr Leu Gln Ile Val Ala Glu Arg Glu Ala Tyr Tyr
    1655                1660                1665

Leu Trp Lys Gln Tyr Asn Pro Thr Gly Lys Gly Ile Asp Asp Ala
    1670                1675                1680

Asn Lys Lys Ala Cys Cys Ala Ile Arg Gly Ser Phe Tyr Asp Leu
    1685                1690                1695

Glu Asp Ile Ile Lys Gly Asn Asp Leu Val His Asp Glu Tyr Thr
    1700                1705                1710

Lys Tyr Ile Asp Ser Lys Leu Asn Glu Ile Phe Gly Ser Ser Asn
    1715                1720                1725

Thr Asn Asp Ile Asp Thr Lys Arg Ala Arg Thr Asp Trp Trp Glu
    1730                1735                1740

Asn Glu Thr Ile Thr Asn Gly Thr Asp Arg Lys Thr Ile Arg Gln
    1745                1750                1755

Leu Val Trp Asp Ala Met Gln Ser Gly Val Arg Tyr Ala Val Glu
    1760                1765                1770

Glu Lys Asn Glu Asn Phe Pro Leu Cys Met Gly Val Glu His Ile
    1775                1780                1785

Gly Ile Ala Lys Pro Gln Phe Ile Arg Trp Leu Glu Glu Trp Thr
    1790                1795                1800

Asn Glu Phe Cys Glu Lys Tyr Thr Lys Tyr Phe Glu Asp Met Lys
    1805                1810                1815

Ser Lys Cys Asp Pro Pro Lys Arg Ala Asp Thr Cys Gly Asp Asn
    1820                1825                1830

Ser Asn Ile Glu Cys Lys Lys Ala Cys Ala Asn Tyr Thr Asn Trp
    1835                1840                1845

Leu Asn Pro Lys Arg Ile Glu Trp Asn Gly Met Ser Asn Tyr Tyr
    1850                1855                1860

Asn Lys Ile Tyr Arg Lys Ser Asn Lys Glu Ser Glu Asp Gly Lys
    1865                1870                1875

Asp Tyr Ser Met Ile Met Ala Pro Thr Val Ile Asp Tyr Leu Asn
    1880                1885                1890

Lys Arg Cys His Gly Glu Ile Asn Gly Asn Tyr Ile Cys Cys Ser
    1895                1900                1905

Cys Lys Asn Ile Gly Ala Tyr Asn Thr Thr Ser Gly Thr Val Asn
    1910                1915                1920

Lys Lys Leu Gln Lys Lys Glu Thr Glu Cys Glu Glu Glu Lys Gly
    1925                1930                1935

Pro Leu Asp Leu Met Asn Glu Val Leu Asn Lys Met Asp Lys Lys
    1940                1945                1950

Tyr Ser Ala His Lys Met Lys Cys Thr Glu Val Tyr Leu Glu His
    1955                1960                1965

Val Glu Glu Gln Leu Asn Glu Ile Asp Asn Ala Ile Lys Asp Tyr
    1970                1975                1980

Lys Leu Tyr Pro Leu Asp Arg Cys Phe Asp Asp Gln Thr Lys Met
    1985                1990                1995

Lys Val Cys Asp Leu Ile Ala Asp Ala Ile Gly Cys Lys Asp Lys
    2000                2005                2010

Thr Lys Leu Asp Glu Leu Asp Glu Trp Asn Asp Met Asp Leu Arg
    2015                2020                2025

Gly Thr Tyr Asn Lys His Lys Gly Val Leu Ile Pro Pro Arg Arg
    2030                2035                2040

Arg Gln Leu Cys Phe Ser Arg Ile Val Arg Gly Pro Ala Asn Leu
    2045                2050                2055

Arg Ser Leu Asn Glu Phe Lys Glu Glu Ile Leu Lys Gly Ala Gln
    2060                2065                2070

Ser Glu Gly Lys Phe Leu Gly Asn Tyr Tyr Lys Glu His Lys Asp
    2075                2080                2085

Lys Glu Lys Ala Leu Glu Ala Met Lys Asn Ser Phe Tyr Asp Tyr
    2090                2095                2100

Glu Asp Ile Ile Lys Gly Thr Asp Met Leu Thr Asn Ile Glu Phe

2105                2110                2115

Lys Asp Ile Lys Ile Lys Leu Asp Arg Leu Leu Glu Lys Glu Thr
    2120                2125                2130

Asn Asn Thr Lys Lys Ala Glu Asp Trp Trp Lys Thr Asn Lys Lys
    2135                2140                2145

Ser Ile Trp Asn Ala Met Leu Cys Gly Tyr Lys Lys Ser Gly Asn
    2150                2155                2160

Lys Ile Ile Asp Pro Ser Trp Cys Thr Ile Pro Thr Thr Glu Thr
    2165                2170                2175

Pro Pro Gln Phe Leu Arg Trp Ile Lys Glu Trp Gly Thr Asn Val
    2180                2185                2190

Cys Ile Gln Lys Gln Glu His Lys Glu Tyr Val Lys Ser Lys Cys
    2195                2200                2205

Ser Asn Val Thr Asn Leu Gly Ala Gln Ala Ser Glu Ser Asn Asn
    2210                2215                2220

Cys Thr Ser Glu Ile Lys Lys Tyr Gln Glu Trp Ser Arg Lys Arg
    2225                2230                2235

Ser Ile Gln Trp Glu Thr Ile Ser Lys Arg Tyr Lys Lys Tyr Lys
    2240                2245                2250

Arg Met Asp Ile Leu Lys Asp Val Lys Glu Pro Asp Ala Asn Thr
    2255                2260                2265

Tyr Leu Arg Glu His Cys Ser Lys Cys Pro Cys Gly Phe Asn Asp
    2270                2275                2280

Met Glu Glu Met Asn Asn Asn Glu Asp Asn Glu Lys Glu Ala Phe
    2285                2290                2295

Lys Gln Ile Lys Glu Gln Val Lys Ile Pro Ala Glu Leu Glu Asp
    2300                2305                2310

Val Ile Tyr Arg Ile Lys His His Glu Tyr Asp Lys Gly Asn Asp
    2315                2320                2325

Tyr Ile Cys Asn Lys Tyr Lys Asn Ile His Asp Arg Met Lys Lys
    2330                2335                2340

Asn Asn Gly Asn Phe Val Thr Asp Asn Phe Val Lys Lys Ser Trp
    2345                2350                2355

Glu Ile Ser Asn Gly Val Leu Ile Pro Pro Arg Arg Lys Asn Leu
    2360                2365                2370

Phe Leu Tyr Ile Asp Pro Ser Lys Ile Cys Glu Tyr Lys Lys Asp
    2375                2380                2385

Pro Lys Leu Phe Lys Asp Phe Ile Tyr Trp Ser Ala Phe Thr Glu
    2390                2395                2400

Val Glu Arg Leu Lys Lys Ala Tyr Gly Gly Ala Arg Ala Lys Val
    2405                2410                2415

Val His Ala Met Lys Tyr Ser Phe Thr Asp Ile Gly Ser Ile Ile
    2420                2425                2430

Lys Gly Asp Asp Met Met Glu Lys Asn Ser Ser Asp Lys Ile Gly
    2435                2440                2445

Lys Ile Leu Gly Asp Thr Asp Gly Gln Asn Glu Lys Arg Lys Lys
    2450                2455                2460

Trp Trp Asp Met Asn Lys Tyr His Ile Trp Glu Ser Met Leu Cys
    2465                2470                2475

Gly Tyr Arg Glu Ala Glu Gly Asp Thr Glu Thr Asn Glu Asn Cys
    2480                2485                2490

Arg Phe Pro Asp Ile Glu Ser Val Pro Gln Phe Leu Arg Trp Phe
    2495                2500                2505

Gln Glu Trp Ser Glu Asn Phe Cys Asp Arg Arg Gln Lys Leu Tyr
    2510                2515                2520

Asp Lys Leu Asn Ser Glu Cys Ile Ser Ala Glu Cys Thr Asn Gly
    2525                2530                2535

Ser Val Asp Asn Ser Lys Cys Thr His Ala Cys Val Asn Tyr Lys
    2540                2545                2550

Asn Tyr Ile Leu Thr Lys Lys Thr Glu Tyr Glu Ile Gln Thr Asn
    2555                2560                2565

Lys Tyr Asp Asn Glu Phe Lys Asn Lys Asn Ser Asn Asp Lys Asp
    2570                2575                2580

Ala Pro Asp Tyr Leu Lys Glu Lys Cys Asn Asp Asn Lys Cys Glu
    2585                2590                2595

Cys Leu Asn Lys His Ile Asp Asp Lys Asn Lys Thr Trp Lys Asn
    2600                2605                2610

Pro Tyr Glu Thr Leu Glu Asp Thr Phe Lys Ser Lys Cys Asp Cys
    2615                2620                2625

Pro Lys Pro Leu Pro Ser Pro Ile Lys Pro Asp Asp Leu Pro Pro
    2630                2635                2640

Gln Ala Asp Glu Pro Phe
    2645

<210> 6
<211> 7947
<212> DNA
<213> artificial sequence

<220>
<223> nucleotide sequence encoding VAR2CSA protein of Plasmodium falciparum
strain pf FCR3

<400> 6
atggacagca ccagcacaat cgccaacaag atcgaggaat atctgggagc taagtccgat 60

gacagcaaga tcgacgaact gctgaaggcc gatcctagcg aagtggagta ctacagaagc 120

ggaggcgacg gcgactacct gaagaacaac atctgcaaga tcaccgtgaa ccacagcgat 180

agcggcaagt atgacccctg cgagaagaag ctgcccccct acgacgacaa cgaccagtgg 240

aagtgccagc agaacagcag cgacggcagc ggcaagcccg agaacatctg cgtgcccccc 300

agacgggagc ggctgtgcac ctacaacctg gaaaacctga agttcgacaa gatccgggac 360

aacaacgcct tcctggccga cgtgctgctg accgcccgga acgagggcga gaagatcgtg 420

cagaaccacc ccgacaccaa cagcagcaac gtgtgcaacg ccctggaacg gtccttcgct 480

gacctggctg acatcatccg gggcaccgat cagtggaagg gcaccaactc caatctggaa 540

aagaacctga agcagatgtt cgccaagatc agagaaaacg acaaggtgct gcaggacaag 600

taccccaagg accagaagta caccaagctg cgggaggcct ggtggaacgc caaccggcag 660

aaagtgtggg aagtgatcac ctgtggcgcc agaagcaacg atctgctgat caagcggggc 720

tggcggacca gcggcaagag cgaccggaag aaaaacttcg agctgtgccg gaagtgcggc 780

cactacgaga aagaggtgcc caccaagctg gactacgtgc cccagttcct gcggtggctg 840

accgagtgga tcgaggactt ctaccgggag aagcagaacc tgatcgacga catggaacgg 900

caccgggagg aatgcaccag agaggaccac aagagcaaag agggcaccag ctactgcagc 960

acatgcaagg acaagtgcaa gaaatactgc gagtgcgtga agaaatggaa aaccgagtgg 1020

gagaaccagg aaaacaagta caaggacctg tacgagcaga acaagaacaa gaccagccag 1080

aagaacacca gcagatacga cgactacgtg aaggacttct tcgagaagct ggaagccaac 1140

tacagcagcc tggaaaacta catcaagggc gacccctatt tcgctgagta cgctacaaaa 1200

ctgagcttca tcctgaaccc cagcgacgcc aacaacccca gcggcgagac agccaaccac 1260

aacgacgagg cctgcaactg caacgagagc ggcatcagca gcgtgggcca ggctcagaca 1320

tccggcccta gcagcaacaa gacctgtatc acccacagct ccatcaagac caacaagaaa 1380

aaagaatgca aggacgtgaa gctgggcgtg cgggagaacg acaaggatct gaagatctgc 1440

gtgatcgagg acaccagcct gagcggcgtg gacaactgct gctgccagga tctgctgggc 1500

atcctgcagg aaaactgcag cgacaacaag cggggcagca gctccaacga cagctgcgac 1560

aataagaacc aggacgagtg ccagaaaaag ctggaaaagg tgttcgccag cctgaccaac 1620

ggctacaagt gcgataagtg caagagcggc acctcccggt ccaagaagaa gtggatctgg 1680

aagaagtcca gcggcaacga ggaaggcctg caggaagagt acgccaacac catcggcctg 1740

ccccccagga cccagagcct gtacctgggc aatctgccca aactggaaaa cgtgtgcgag 1800

gatgtgaagg acatcaactt cgacaccaaa gagaagtttc tggccggctg cctgatcgtg 1860

tccttccacg agggcaagaa tctgaagaag cgctaccccc agaataagaa cagcggcaac 1920

aaagaaaacc tgtgcaaggc tctggaatac agcttcgccg actacggcga cctgatcaag 1980

ggcacctcca tctgggacaa cgagtacaca aaggacctgg aactgaatct gcagaacaac 2040

ttcggcaagc tgttcggcaa gtacatcaag aagaacaata ccgccgagca ggacacctcc 2100

tacagctccc tggacgagct gcgcgagtct tggtggaata ccaataagaa gtacatctgg 2160

accgccatga agcacggcgc cgagatgaac atcaccacct gtaacgccga cggctccgtg 2220

accggcagcg gctccagctg cgacgacatc cccaccatcg acctgatccc ccagtacctg 2280

agatttctgc aggaatgggt cgagaacttc tgcgagcagc ggcaggccaa agtgaaggac 2340

gtgatcacca actgcaagag ctgcaaagaa tccggcaaca aatgcaagac cgagtgcaaa 2400

accaagtgca aggatgagtg cgagaagtac aagaagttca tcgaggcctg cggcacagcc 2460

ggcggaggca tcggaacagc cggcagcccc tggtccaaga gatgggacca gatctacaag 2520

cggtacagca agcacatcga ggacgccaag cggaaccgga aggccggcac caagaactgc 2580

ggcaccagct ccaccaccaa cgccgctgcc agcaccgacg agaataagtg cgtgcagagc 2640

gacatcgaca gctttttcaa gcacctgatc gatatcggcc tgaccacccc cagcagctac 2700

ctgagcaacg tgctggacga caacatctgt ggcgccgaca aggccccctg gacaacctat 2760

acaacataca ctacaaccga gaagtgcaac aaagagcggg acaagagcaa gagccagagc 2820

agcgacaccc tggtggtggt gaacgtgccc agccccctgg gcaacacacc ctaccggtac 2880

aagtacgcct gccagtgcaa gatccccacc aacgaggaaa catgcgacga ccggaaagaa 2940

tacatgaacc agtggtcctg cgggagcgct cggaccatga agagagggta taagaacgat 3000

aactacgaac tgtgcaagta caacggcgtg gatgtgaagc ccaccaccgt gcggagcaac 3060

tccagcaagc tggacggcaa cgacgtgacc ttcttcaacc tgttcgagca gtggaacaaa 3120

gaaatccagt accagatcga gcagtacatg accaacgcca acatcagctg catcgatgag 3180

aaagaagtgc tggacagcgt ctccgacgag ggcaccccca aagtgcgggg aggctacgag 3240

gacggccgga acaacaacac agatcagggc acaaattgca aagaaaagtg caagtgctac 3300

aagctgtgga tcgagaagat caacgatcag tggggcaagc agaaggacaa ctacaacaag 3360

ttccggtcca agcagatcta cgacgccaat aagggcagcc agaacaaaaa ggtggtgtcc 3420

ctgagcaact tcctgttctt cagctgctgg gaggaataca tccagaagta cttcaacggc 3480

gattggagca agatcaagaa catcggctcc gacaccttcg agtttctgat caagaagtgc 3540

ggcaacaaca gcgcccacgg cgaggaaatc ttcagcgaga agctgaagaa cgccgagaag 3600

aagtgcaaag agaacgagag caccgacacc aatatcaaca agagcgagac atcctgcgac 3660

ctgaacgcca ccaactacat ccggggctgc cagagcaaga cctacgacgg caagatcttc 3720

cccggcaagg gcggcgagaa gcagtggatt tgcaaggaca ccatcatcca cggcgacacc 3780

aacggcgcct gcatcccccc tcgcacccag aacctgtgcg tgggcgagct gtgggacaag 3840

tcctacggcg gcagatccaa catcaagaac gataccaaag aactgctgaa agagaagatt 3900

aagaacgcca tccacaaaga gacagagctg ctgtacgagt accacgacac cggcaccgcc 3960

atcatcagca agaatgacaa gaagggccag aagggcaaga acgaccctaa cggcctgccc 4020

aaaggcttct gtcatgctgt gcagcggagc ttcatcgact acaagaacat gatcctgggc 4080

accagcgtga acatctacga gcacatcggc aagctgcagg aagatatcaa gaagatcatc 4140

gaaaagggca cccctcagca gaaggataag atcggcggcg tgggcagcag caccgagaac 4200

gtgaacgctt ggtggaaggg aatcgagcgg gagatgtggg acgccgtgag atgcgccatc 4260

accaagatca ataagaagaa taacaacagc atcttcaatg gcgacgagtg cggcgtgagc 4320

ccccccaccg gcaacgatga ggaccagagc gtgtcctggt tcaaagagtg gggcgagcag 4380

ttctgcatcg agcggctgag atacgaacag aacatcagag aggcctgcac catcaacggc 4440

aaaaacgaga agaaatgcat caacagcaag tccggccagg gcgataagat ccagggcgcc 4500

tgcaagcgga agtgtgaaaa gtataagaag tatatcagcg agaaaaaaca ggaatgggac 4560

aagcagaaaa ccaagtacga gaacaaatac gtgggcaaga gcgccagcga tctgctgaaa 4620

gaaaactacc ccgagtgcat cagcgccaac ttcgacttca tcttcaacga caatatcgag 4680

tacaagacct actaccccta cggcgattac agcagcatct gcagctgcga acaggtgaag 4740

tactataagt acaacaatgc cgagaaaaag aacaacaaga gcctgtgcta cgagaaggac 4800

aatgacatga cctggtctaa gaaatatatc aagaagctgg aaaacggccg gtccctggaa 4860

ggcgtgtacg tgccccctcg gcggcagcag ctgtgtctgt acgagctgtt ccctatcatc 4920

atcaaaaacg aagagggcat ggaaaaggcc aaagaggaac tgctggaaac cctgcagatc 4980

gtggccgagc gcgaggccta ctacctgtgg aagcagtaca accccacagg caaagggatt 5040

gatgatgcca acaagaaggc ctgctgcgcc atcagaggca gcttctacga cctggaagat 5100

attatcaagg gcaacgacct ggtgcacgac gagtacacca aatacatcga ctccaagctg 5160

aacgagatct tcggctccag caacaccaac gacatcgata ccaagcgggc cagaaccgat 5220

tggtgggaga acgagacaat caccaacggc accgacagaa agaccatccg gcagctggtg 5280

tgggatgcca tgcagagcgg agtgcgatat gctgtggagg aaaagaacga gaacttcccc 5340

ctgtgcatgg gcgtggagca cattggcatt gccaagcccc agttcatcag atggctggaa 5400

gagtggacca acgagttctg cgagaaatac accaagtact tcgaggacat gaagtccaag 5460

tgcgaccccc ccaagagggc cgacacatgc ggcgacaact ccaacatcga gtgcaagaag 5520

gcttgcgcca actacaccaa ctggctgaac cccaagcgga tcgagtggaa cggcatgagc 5580

aactactaca ataagatcta ccggaagtct aacaaagaga gcgaggacgg caaggactac 5640

agcatgatca tggcccccac cgtgatcgat tacctgaaca agcggtgcca cggcgagatc 5700

aatggcaact acatctgctg cagctgcaag aatatcggcg cctacaacac cacctccggc 5760

accgtgaaca agaagctgca gaaaaaagaa accgagtgcg aggaagagaa gggccctctg 5820

gacctgatga acgaggtgct gaacaagatg gacaagaagt actccgccca caagatgaag 5880

tgcaccgagg tgtacctgga acacgtggag gaacagctga atgagatcga caacgccatc 5940

aaggattaca agctgtaccc cctggacaga tgcttcgacg accagaccaa gatgaaagtg 6000

tgcgatctga tcgccgacgc catcggctgc aaggataaga caaagctgga tgagctggac 6060

gaatggaacg acatggacct gagaggcacc tacaataagc acaagggcgt gctgatcccc 6120

cccaggcgga gacagctgtg cttcagccgg atcgtgcggg gacccgccaa cctgagaagc 6180

ctgaacgagt tcaaagagga aatcctgaag ggcgctcagt ccgagggcaa gttcctgggc 6240

aattactaca aagagcacaa ggacaaagag aaggccctgg aagccatgaa gaacagcttt 6300

tacgactacg aggacatcat caagggaacc gacatgctga ccaacatcga attcaaggat 6360

atcaagatca agctggaccg gctgctggaa aaagagacaa acaacaccaa gaaagccgag 6420

gactggtgga aaaccaacaa aaagtctatt tggaacgcca tgctgtgcgg ctacaaaaag 6480

agcggcaata agatcattga ccccagctgg tgcaccatcc ctaccaccga gacacccccc 6540

cagtttctga gatggatcaa agaatggggc accaatgtgt gcatccagaa gcaggaacac 6600

aaagaatacg tcaagtctaa gtgctccaac gtgaccaatc tgggagcaca ggccagcgag 6660

agcaacaact gcaccagcga gatcaagaaa taccaggaat ggtcccggaa gcggagcatc 6720

cagtgggaga caatcagcaa gcgctacaag aaatacaagc ggatggacat cctgaaggat 6780

gtgaaagagc ctgacgccaa tacctacctg agagaacact gcagcaagtg cccctgcggc 6840

ttcaacgata tggaagagat gaacaacaac gaggacaatg agaaagaggc cttcaagcag 6900

atcaaagaac aggtgaaaat ccccgccgag ctggaagatg tgatctacag aatcaagcac 6960

cacgagtacg acaagggcaa tgactacatc tgtaacaaat acaagaatat ccacgaccgg 7020

atgaagaaaa acaacggcaa cttcgtgacc gacaatttcg tgaagaagtc ctgggagatt 7080

tccaatggag tgctgatccc tcctcggaga aagaacctgt tcctgtacat cgaccccagc 7140

aagatctgcg aatacaagaa ggaccccaag ctgttcaagg acttcatcta ttggagcgcc 7200

ttcaccgagg tggagcggct gaagaaggcc tacggcggag ccagagccaa ggtggtgcac 7260

gccatgaagt atagcttcac cgacatcggc agcatcatta agggcgacga catgatggaa 7320

aagaatagca gcgacaagat cggcaagatc ctgggggaca ccgacggcca gaacgagaag 7380

cgcaaaaagt ggtgggacat gaacaagtac cacatctggg agagcatgct gtgtggatac 7440

cgggaggccg agggagatac agagactaac gaaaactgcc ggttccccga catcgagagc 7500

gtgcctcagt ttctgcgctg gtttcaggaa tggtcagaga atttttgcga tagaaggcag 7560

aagctgtacg acaagctgaa cagcgagtgc atctccgccg agtgcaccaa tggctccgtg 7620

gacaatagca agtgcaccca cgcctgcgtg aactacaaga attacatcct gaccaaaaag 7680

accgagtacg agatccagac caataagtac gacaacgagt ttaagaacaa gaatagcaac 7740

gataaggacg cccccgacta tctgaaagag aaatgcaacg acaacaagtg cgagtgcctg 7800

aataagcaca ttgacgacaa gaacaaaacc tggaagaacc cctacgagac actggaagat 7860

accttcaaga gcaagtgcga ctgccctaag cccctgccct cccccatcaa gcccgacgat 7920

ctgccacccc aggccgacga gcccttc 7947

<210> 7
<211> 632
<212> PRT
<213> artificial sequence

<220>
<223> amino acid sequence of the peptide fragment ID1-ID2a

<400> 7

Asn Tyr Ile Lys Gly Asp Pro Tyr Phe Ala Glu Tyr Ala Thr Lys Leu
1               5                   10                  15

Ser Phe Ile Leu Asn Ser Ser Asp Ala Asn Asn Pro Ser Glu Lys Ile
            20                  25                  30

Gln Lys Asn Asn Asp Glu Val Cys Asn Cys Asn Glu Ser Gly Ile Ala
        35                  40                  45

Ser Val Glu Gln Glu Gln Ile Ser Asp Pro Ser Ser Asn Lys Thr Cys
    50                  55                  60

Ile Thr His Ser Ser Ile Lys Ala Asn Lys Lys Lys Val Cys Lys His
65                  70                  75                  80

Val Lys Leu Gly Val Arg Glu Asn Asp Lys Asp Leu Arg Val Cys Val
                85                  90                  95

Ile Glu His Thr Ser Leu Ser Gly Val Glu Asn Cys Cys Cys Gln Asp
            100                 105                 110

Phe Leu Arg Ile Leu Gln Glu Asn Cys Ser Asp Asn Lys Ser Gly Ser
        115                 120                 125

Ser Ser Asn Gly Ser Cys Asn Asn Lys Asn Gln Glu Ala Cys Glu Lys
    130                 135                 140

Asn Leu Glu Lys Val Leu Ala Ser Leu Thr Asn Cys Tyr Lys Cys Asp
145                 150                 155                 160

Lys Cys Lys Ser Glu Gln Ser Lys Lys Asn Asn Lys Asn Trp Ile Trp
                165                 170                 175

Lys Lys Ser Ser Gly Lys Glu Gly Gly Leu Gln Lys Glu Tyr Ala Asn
            180                 185                 190

Thr Ile Gly Leu Pro Pro Arg Thr Gln Ser Leu Cys Leu Val Val Cys
        195                 200                 205

Leu Asp Glu Lys Gly Lys Lys Thr Gln Glu Leu Lys Asn Ile Arg Thr
    210                 215                 220

Asn Ser Glu Leu Leu Lys Glu Trp Ile Ile Ala Ala Phe His Glu Gly
225                 230                 235                 240

Lys Asn Leu Lys Pro Ser His Glu Lys Lys Asn Asp Asp Asn Gly Lys
                245                 250                 255

Lys Leu Cys Lys Ala Leu Glu Tyr Ser Phe Ala Asp Tyr Gly Asp Leu
            260                 265                 270

Ile Lys Gly Thr Ser Ile Trp Asp Asn Glu Tyr Thr Lys Asp Leu Glu
        275                 280                 285

Leu Asn Leu Gln Lys Ile Phe Gly Lys Leu Phe Arg Lys Tyr Ile Lys
    290                 295                 300

Lys Asn Asn Thr Ala Glu Gln Asp Thr Ser Tyr Ser Ser Leu Asp Glu
305                 310                 315                 320

Leu Arg Glu Ser Trp Trp Asn Thr Asn Lys Lys Tyr Ile Trp Leu Ala
                325                 330                 335

Met Lys His Gly Ala Gly Met Asn Ser Thr Thr Cys Cys Gly Asp Gly
            340                 345                 350

Ser Val Thr Gly Ser Gly Ser Ser Cys Asp Asp Ile Pro Thr Ile Asp
        355                 360                 365

Leu Ile Pro Gln Tyr Leu Arg Phe Leu Gln Glu Trp Val Glu His Phe
    370                 375                 380

Cys Lys Gln Arg Gln Glu Lys Val Lys Pro Val Ile Glu Asn Cys Lys
385                 390                 395                 400

Ser Cys Lys Glu Ser Gly Gly Thr Cys Asn Gly Glu Cys Lys Thr Glu
                405                 410                 415

Cys Lys Asn Lys Cys Glu Val Tyr Lys Lys Phe Ile Glu Asp Cys Lys
            420                 425                 430

Gly Gly Asp Gly Thr Ala Gly Ser Ser Trp Val Lys Arg Trp Asp Gln
        435                 440                 445

Ile Tyr Lys Arg Tyr Ser Lys Tyr Ile Glu Asp Ala Lys Arg Asn Arg
    450                 455                 460

Lys Ala Gly Thr Lys Asn Cys Gly Pro Ser Ser Thr Thr Asn Ala Ala
465                 470                 475                 480

Glu Asn Lys Cys Val Gln Ser Asp Ile Asp Ser Phe Phe Lys His Leu
                485                 490                 495

Ile Asp Ile Gly Leu Thr Thr Pro Ser Ser Tyr Leu Ser Ile Val Leu
            500                 505                 510

Asp Asp Asn Ile Cys Gly Ala Asp Lys Ala Pro Trp Thr Thr Tyr Thr
        515                 520                 525

Thr Tyr Thr Thr Thr Glu Lys Cys Asn Lys Glu Thr Asp Lys Ser Lys

530                 535                 540

Leu Gln Gln Cys Asn Thr Ala Val Val Val Asn Val Pro Ser Pro Leu
545                 550                 555                 560

Gly Asn Thr Pro His Gly Tyr Lys Tyr Ala Cys Gln Cys Lys Ile Pro
                565                 570                 575

Thr Asn Glu Glu Thr Cys Asp Asp Arg Lys Glu Tyr Met Asn Gln Trp
            580                 585                 590

Ser Cys Gly Ser Ala Arg Thr Met Lys Arg Gly Tyr Lys Asn Asp Asn
        595                 600                 605

Tyr Glu Leu Cys Lys Tyr Asn Gly Val Asp Val Lys Pro Thr Thr Val
    610                 615                 620

Arg Ser Asn Ser Ser Lys Leu Asp
625                 630

<210>  8
<211>  1896
<212>  DNA
<213>  artificial sequence

<220>
<223> nucleotide sequence encoding the peptide fragment ID1-ID2a

<400>  8
aactacatca agggcgaccc ctacttcgcc gagtacgcca ccaagctgag cttcatcctg      60

aacagcagcg acgccaacaa ccccagcgag aagatccaga agaacaacga cgaggtgtgc     120

aactgcaacg agagcggcat cgccagcgtg gagcaggagc agatcagcga ccccagcagc     180

aacaagacct gcatcaccca cagcagcatc aaggccaaca agaagaaggt gtgcaagcac     240

gtgaagctgg gcgtgcgcga gaacgacaag gacctgcgcg tgtgcgtgat cgagcacacc     300

agcctgagcg gcgtggagaa ctgctgctgc caggacttcc tgcgcatcct gcaggagaac     360

tgcagcgaca acaagagcgg cagcagcagc aacggcagct gcaacaacaa gaaccaggag     420

gcctgcgaga agaacctgga gaaggtgctg gccagcctga ccaactgcta caagtgcgac     480

aagtgcaaga gcgagcagag caagaagaac aacaagaact ggatctggaa gaagagcagc     540

ggcaaggagg gcggcctgca gaaggagtac gccaacacca tcggcctgcc cccccgcacc      600

cagagcctgt gcctggtggt gtgcctggac gagaagggca agaagaccca ggagctgaag     660

aacatccgca ccaacagcga gctgctgaag gagtggatca tcgccgcctt ccacgagggc     720

aagaacctga agcccagcca cgagaagaag aacgacgaca acggcaagaa gctgtgcaag     780

gccctggagt acagcttcgc cgactacggc gacctgatca agggcaccag catctgggac     840

aacgagtaca ccaaggacct ggagctgaac ctgcagaaga tcttcggcaa gctgttccgc     900

aagtacatca agaagaacaa caccgccgag caggacacca gctacagcag cctggacgag     960

ctgcgcgaga gctggtggaa caccaacaag aagtacatct ggctggccat gaagcacggc    1020

gccggcatga acagcaccac ctgctgcggc gacggcagcg tgaccggcag cggcagcagc    1080

tgcgacgaca tccccaccat cgacctgatc ccccagtacc tgcgcttcct gcaggagtgg    1140

gtggagcact tctgcaagca gcgccaggag aaggtgaagc ccgtgatcga gaactgcaag    1200

agctgcaagg agagcggcgg cacctgcaac ggcgagtgca agaccgagtg caagaacaag    1260

tgcgaggtgt acaagaagtt catcgaggac tgcaagggcg gcgacggcac cgccggcagc    1320

agctgggtga agcgctggga ccagatctac aagcgctaca gcaagtacat cgaggacgcc    1380

aagcgcaacc gcaaggccgg caccaagaac tgcggcccca gcagcaccac caacgccgcc    1440

gagaacaagt gcgtgcagag cgacatcgac agcttcttca agcacctgat cgacatcggc    1500

ctgaccaccc ccagcagcta cctgagcatc gtgctggacg acaacatctg cggcgccgac    1560

aaggccccct ggaccaccta caccacctac accaccaccg agaagtgcaa caaggagacc    1620

gacaagagca agctgcagca gtgcaacacc gccgtggtgg tgaacgtgcc cagccccctg    1680

ggcaacaccc cccacggcta caagtacgcc tgccagtgca agatccccac caacgaggag    1740

acctgcgacg accgcaagga gtacatgaac cagtggagct gcggcagcgc ccgcaccatg    1800

aagcgcggct acaagaacga caactacgag ctgtgcaagt acaacggcgt ggacgtgaag    1860

cccaccaccg tgcgcagcaa cagcagcaag ctggac                              1896

<210>  9
<211>  45
<212>  PRT
<213>  artificial sequence

<220>
<223> amino acid sequence of the hinge region of human CD8¦Á

<400>  9

Thr Thr Thr Pro Ala Pro Arg Pro Pro Thr Pro Ala Pro Thr Ile Ala
1               5                   10                  15

Ser Gln Pro Leu Ser Leu Arg Pro Glu Ala Cys Arg Pro Ala Ala Gly
            20                  25                  30

Gly Ala Val His Thr Arg Gly Leu Asp Phe Ala Cys Asp
        35                  40                  45

<210>  10
<211>  135
<212>  DNA

<213>  artificial sequence

<220>
<223> nucleotide sequence encoding the hinge region of human CD8¦Á

<400>  10
accaccaccc ccgcccccg cccccccacc cccgccccca ccatcgccag ccagcccctg      60

agcctgcgcc ccgaggcctg ccgccccgcc gccggcggcg ccgtgcacac ccgcggcctg     120

gacttcgcct gcgac                                                      135


<210>  11
<211>  27
<212>  PRT
<213>  artificial sequence

<220>
<223> amino acid sequence of the transmembrane region of human CD28

<400>  11

Phe Trp Val Leu Val Val Val Gly Gly Val Leu Ala Cys Tyr Ser Leu
1               5                   10                  15


Leu Val Thr Val Ala Phe Ile Ile Phe Trp Val
            20                  25


<210>  12
<211>  81
<212>  DNA
<213>  artificial sequence

<220>
<223>  nucleotide sequence encoding the transmembrane region of human CD28

<400>  12
ttctgggtgc tggtggtggt gggcggcgtg ctggcctgct acagcctgct ggtgaccgtg      60

gccttcatca tcttctgggt g                                                81


<210>  13
<211>  113
<212>  PRT
<213>  artificial sequence

<220>
<223> amino acid sequence of the intracellular region of human CD3¦Æ

<400>  13

Arg Val Lys Phe Ser Arg Ser Ala Asp Ala Pro Ala Tyr Gln Gln Gly
1               5                   10                  15


Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Gln Arg Arg Lys Asn Pro Gln Glu Gly Leu Tyr Asn Glu Leu Gln
    50                  55                  60

Lys Asp Lys Met Ala Glu Ala Tyr Ser Glu Ile Gly Met Lys Gly Glu
65                  70                  75                  80

Arg Arg Arg Gly Lys Gly His Asp Gly Leu Tyr Gln Gly Leu Ser Thr
                85                  90                  95

Ala Thr Lys Asp Thr Tyr Asp Ala Leu His Met Gln Ala Leu Pro Pro
            100                 105                 110

Arg

<210>  14
<211>  339
<212>  DNA
<213>  artificial sequence

<220>
<223> nucleotide sequence encoding the intracellular region of human CD3¦Æ

<400>  14
cgcgtgaagt tcagccgcag cgccgacgcc cccgcctacc agcagggcca gaaccagctg      60

tacaacgagc tgaacctggg ccgccgcgag gagtacgacg tgctggacaa gcgccgcggc     120

cgcgaccccg agatgggcgg caagccccag cgccgcaaga acccccagga gggcctgtac     180

aacgagctgc agaaggacaa gatggccgag gcctacagcg agatcggcat gaagggcgag     240

cgccgccgcg gcaagggcca cgacggcctg taccagggcc tgagcaccgc caccaaggac     300

acctacgacg ccctgcacat gcaggccctg cccccccgc                            339

<210>  15
<211>  154
<212>  PRT
<213>  artificial sequence

<220>
<223> amino acid sequence of the tandem arrangement of the intracellular
region of human CD28 and the intracellular region of human CD3¦Æ

<400>  15

Arg Ser Lys Arg Ser Arg Leu Leu His Ser Asp Tyr Met Asn Met Thr
1               5                   10                  15

Pro Arg Arg Pro Gly Pro Thr Arg Lys His Tyr Gln Pro Tyr Ala Pro
            20                  25                  30

Pro Arg Asp Phe Ala Ala Tyr Arg Ser Arg Val Lys Phe Ser Arg Ser
        35                  40                  45

Ala Asp Ala Pro Ala Tyr Gln Gln Gly Gln Asn Gln Leu Tyr Asn Glu
    50                  55                  60

Leu Asn Leu Gly Arg Arg Glu Glu Tyr Asp Val Leu Asp Lys Arg Arg
65                  70                  75                  80

Gly Arg Asp Pro Glu Met Gly Gly Lys Pro Gln Arg Arg Lys Asn Pro
                85                  90                  95

Gln Glu Gly Leu Tyr Asn Glu Leu Gln Lys Asp Lys Met Ala Glu Ala
            100                 105                 110

Tyr Ser Glu Ile Gly Met Lys Gly Glu Arg Arg Arg Gly Lys Gly His
        115                 120                 125

Asp Gly Leu Tyr Gln Gly Leu Ser Thr Ala Thr Lys Asp Thr Tyr Asp
    130                 135                 140

Ala Leu His Met Gln Ala Leu Pro Pro Arg
145                 150

<210>  16
<211>  462
<212>  DNA
<213>  artificial sequence

<220>
<223> nucleotide sequence encoding the tandem arrangement of the
intracellular region of human CD28 and the intracellular region of human
CD3¦Æ

<400>  16
cgcagcaagc gcagccgcct gctgcacagc gactacatga acatgacccc ccgccgcccc      60

ggccccaccc gcaagcacta ccagccctac gccccccccc gcgacttcgc cgcctaccgc     120

agccgcgtga agttcagccg cagcgccgac gcccccgcct accagcaggg ccagaaccag     180

ctgtacaacg agctgaacct gggccgccgc gaggagtacg acgtgctgga caagcgccgc     240

ggccgcgacc ccgagatggg cggcaagccc cagcgccgca agaaccccca ggagggcctg     300

tacaacgagc tgcagaagga caagatggcc gaggcctaca gcgagatcgg catgaagggc     360

gagcgccgcc gcggcaaggg ccacgacggc ctgtaccagg gcctgagcac cgccaccaag     420

gacacctacg acgccctgca catgcaggcc ctgccccccc gc                        462

<210>  17

<211>  155
<212>  PRT
<213>  artificial sequence

<220>
<223> amino acid sequence of the tandem arrangement of the intracellular
region of human 4-1BB and the intracellular region of human CD3¦Æ

<400>  17

Lys Arg Gly Arg Lys Lys Leu Leu Tyr Ile Phe Lys Gln Pro Phe Met
1               5                   10                  15

Arg Pro Val Gln Thr Thr Gln Glu Glu Asp Gly Cys Ser Cys Arg Phe
            20                  25                  30

Pro Glu Glu Glu Glu Gly Gly Cys Glu Leu Arg Val Lys Phe Ser Arg
        35                  40                  45

Ser Ala Asp Ala Pro Ala Tyr Gln Gln Gly Gln Asn Gln Leu Tyr Asn
    50                  55                  60

Glu Leu Asn Leu Gly Arg Arg Glu Glu Tyr Asp Val Leu Asp Lys Arg
65                  70                  75                  80

Arg Gly Arg Asp Pro Glu Met Gly Gly Lys Pro Gln Arg Arg Lys Asn
                85                  90                  95

Pro Gln Glu Gly Leu Tyr Asn Glu Leu Gln Lys Asp Lys Met Ala Glu
            100                 105                 110

Ala Tyr Ser Glu Ile Gly Met Lys Gly Glu Arg Arg Arg Gly Lys Gly
        115                 120                 125

His Asp Gly Leu Tyr Gln Gly Leu Ser Thr Ala Thr Lys Asp Thr Tyr
    130                 135                 140

Asp Ala Leu His Met Gln Ala Leu Pro Pro Arg
145                 150                 155

<210>  18
<211>  465
<212>  DNA
<213>  artificial sequence

<220>
<223> nucleotide sequence encoding the tandem arrangement of the
intracellular region of human 4-1BB and the intracellular region of human
CD3¦Æ

<400>  18
aagcgcggcc gcaagaagct gctgtacatc ttcaagcagc ccttcatgcg ccccgtgcag      60

accacccagg aggaggacgg ctgcagctgc cgcttccccg aggaggagga gggcggctgc     120

gagctgcgcg tgaagttcag ccgcagcgcc gacgcccccg cctaccagca gggccagaac     180

cagctgtaca acgagctgaa cctgggccgc cgcgaggagt acgacgtgct ggacaagcgc     240

cgcggccgcg accccgagat gggcggcaag ccccagcgcc gcaagaaccc ccaggagggc     300

ctgtacaacg agctgcagaa ggacaagatg gccgaggcct acagcgagat cggcatgaag     360

ggcgagcgcc gccgcggcaa gggccacgac ggcctgtacc agggcctgag caccgccacc     420

aaggacacct acgacgccct gcacatgcag gccctgcccc cccgc                     465


<210>  19
<211>  196
<212>  PRT
<213>  artificial sequence

<220>
<223> amino acid sequence of the tandem arrangement of the intracellular
region of human CD28, the intracellular region of human 4-1BB and the
intracellular region of human CD3¦Æ

<400>  19

Arg Ser Lys Arg Ser Arg Leu Leu His Ser Asp Tyr Met Asn Met Thr
1               5                   10                  15


Pro Arg Arg Pro Gly Pro Thr Arg Lys His Tyr Gln Pro Tyr Ala Pro
            20                  25                  30


Pro Arg Asp Phe Ala Ala Tyr Arg Ser Lys Arg Gly Arg Lys Lys Leu
        35                  40                  45


Leu Tyr Ile Phe Lys Gln Pro Phe Met Arg Pro Val Gln Thr Thr Gln
    50                  55                  60


Glu Glu Asp Gly Cys Ser Cys Arg Phe Pro Glu Glu Glu Glu Gly Gly
65                  70                  75                  80


Cys Glu Leu Arg Val Lys Phe Ser Arg Ser Ala Asp Ala Pro Ala Tyr
                85                  90                  95


Gln Gln Gly Gln Asn Gln Leu Tyr Asn Glu Leu Asn Leu Gly Arg Arg
            100                 105                 110


Glu Glu Tyr Asp Val Leu Asp Lys Arg Arg Gly Arg Asp Pro Glu Met
        115                 120                 125


Gly Gly Lys Pro Gln Arg Arg Lys Asn Pro Gln Glu Gly Leu Tyr Asn
    130                 135                 140

Glu Leu Gln Lys Asp Lys Met Ala Glu Ala Tyr Ser Glu Ile Gly Met
145                 150                 155                 160

Lys Gly Glu Arg Arg Arg Gly Lys Gly His Asp Gly Leu Tyr Gln Gly
                165                 170                 175

Leu Ser Thr Ala Thr Lys Asp Thr Tyr Asp Ala Leu His Met Gln Ala
            180                 185                 190

Leu Pro Pro Arg
        195

<210>  20
<211>  588
<212>  DNA
<213>  artificial sequence

<220>
<223> nucleotide sequence encoding the tandem arrangement of the
intracellular region of human CD28, the intracellular region of human 4-1BB
and the intracellular region of human CD3¦Æ

<400>  20
cgcagcaagc gcagccgcct gctgcacagc gactacatga acatgacccc ccgccgcccc      60

ggccccaccc gcaagcacta ccagccctac gccccccccc gcgacttcgc cgcctaccgc     120

agcaagcgcg gccgcaagaa gctgctgtac atcttcaagc agcccttcat gcgccccgtg     180

cagaccaccc aggaggagga cggctgcagc tgccgcttcc ccgaggagga ggagggcggc     240

tgcgagctgc gcgtgaagtt cagccgcagc gccgacgccc ccgcctacca gcagggccag     300

aaccagctgt acaacgagct gaacctgggc cgccgcgagg agtacgacgt gctggacaag     360

cgccgcggcc gcgaccccga gatgggcggc aagccccagc gccgcaagaa cccccaggag     420

ggcctgtaca acgagctgca gaaggacaag atggccgagg cctacagcga gatcggcatg     480

aagggcgagc gccgccgcgg caagggccac gacggcctgt accagggcct gagcaccgcc     540

accaaggaca cctacgacgc cctgcacatg caggccctgc ccccccgc                  588

<210>  21
<211>  21
<212>  PRT
<213>  artificial sequence

<220>
<223> amino acid sequence of the human CD8¦Á signal peptide

<400>  21

Met Ala Leu Pro Val Thr Ala Leu Leu Leu Pro Leu Ala Leu Leu Leu
1               5                   10                  15

His Ala Ala Arg Pro

            20

<210> 22
<211> 63
<212> DNA
<213> CD8¦Á

<400> 22
atggccttac cagtgaccgc cttgctcctg ccgctggcct tgctgctcca cgccgccagg 60

ccg 63

## Description

### Technical Field

The present invention relates to the field of cellular immunotherapy for tumors, in particular to a chimeric antigen receptor of anti-placental-like chondroitin sulfate and application thereof, specifically to a construction method of chimeric antigen receptor T cell (CAR-T) technology based on a tumor-specific target pl-CS and its application in anti-tumor therapy.

### Background

The chimeric antigen receptor T cell (CAR-T) technology is a novel cell therapy that is currently undergoing large-scale clinical trials. It has significant efficiency on the treatment of acute leukemia and non-Hodgkin's lymphoma, and is considered as one of the most promising methods for treating tumors. The basic technique thereof is to transform T cells into those that recognize tumor antigens in a HLA-independent manner through genetic engineering methods, allowing CAR-T cells to have stronger ability to recognize and kill tumor cells than natural T cells. The core component of CAR-T is the CAR, which typically includes: a tumor-associated antigen binding region typically derived from scFv fragment of a monoclonal antibody against the tumor-associated antigen; an extracellular hinge region; a transmembrane region and an intracellular stimulation signal region.

The key to the success of CAR-T technology is the selection of tumor antigens, *i.e*. the targets. The ideal antigen is an antigen that is highly expressed on the surface of tumor cells and is not expressed in normal cells. Only by selection of such antigens, CAR-T cells will be enabled to specifically bind to and kill tumor cells, rather than bind to normal cells, and thereby will not impair normal tissues. However, there are few such ideal antigens. Most antigens are highly expressed or over-expressed in tumor tissues and low expressed in normal tissues. At present, the treatment efficiency of CAR-T for solid tumors is far less than that for leukemia, one of the reasons for which is that no ideal targets have been found in solid tumors.

Another reason why CAR-T technology is not effective for treating solid tumors is the existence of a tumor immunosuppressive microenvironment. The tumor immunosuppressive microenvironment is an internal environment formed during the development and progression of a tumor and is composed of tumor cells, mesenchymal cells, immunoregulatory cells, microvessels, interstitial fluid, cytokines, and extracellular matrix, *etc.* Due to the existence of immune microenvironment, immune killer cells cannot reach the tumor cell sites effectively or cannot effectively kill tumor cells after reaching the target sites. To effectively treat solid tumors, CAR-T cells must break through the limits of tumor immune microenvironment and enter solid tumors to kill tumor cells.

CN 104788573 A discloses a chimeric antigen receptor hCD19scFv-CD8α-CD28-CD3 ξ and its use. The chimeric antigen receptor is composed of the variable regions of light and heavy chains of anti-human CD19 monoclonal antibody (hCD19scFv), the hinge region of human CD8α, the transmembrane region, and the intracellular region of human CD28 and the intracellular region of human CD3ζ in tandem arrangement. The chimeric antigen receptor is used to modify T lymphocytes, and the modified T cells (CAR-T cells) can be used to treat tumors with CD19 positive on the surface. The chimeric antigen receptor of this patent is only directed to tumors with CD 19 positive on the surface, and the recognition effect is not well. Therefore, both screening suitable tumor targets and breaking through the tumor microenvironment are the keys to treating solid tumors by CAR-T technology.

Chondroitin sulfate (CS) is a kind of glycosaminoglycan that is covalently attached to proteins to form proteoglycans. Chondroitin sulfate is widely distributed on the extracellular matrix and cell surface of animal tissues, and the sugar chain is composed of disaccharide units of alternating glucuronic acid and N-acetylgalactosamine (also known as N-acetylgalactose), attached to the serine residue of the core protein through a sugar-like linking region. Chondroitin sulfate exists in all organisms other than plants, from nematodes to human beings, and plays an important role in many physiological functions. Although the backbone structure of the polysaccharide is not complicated, it shows a high degree of heterogeneity in terms of the degree of sulfation, the distribution of sulfate groups and the two epimeric uronic acids in the chain. The fine structure of chondroitin sulfate determines the specificity of the function and the interaction with various protein molecules.

There exists a special chondroitin sulfate in the placenta, the glycosylation pattern of which is different from that of conventional chondroitin sulfate. In *Plasmodium falciparum,* there exists a protein that is called VAR2CSA and can specifically bind to the chondroitin sulfate in placenta. On the surface of tumor cells, there exists a placental-like chondroitin sulfate (pl-CS), and therefore, pl-CS is an ideal target for the treatment of tumors with CAR-T technology.

### Summary of the Invention

The present invention provides a chimeric antigen receptor of anti-placental-like chondroitin sulfate and its application in view of the facts that the selection of targets for the treatment of tumors with CAR-T technology is not ideal currently and that the therapeutic effect of CAR-T technology is impaired by tumor microenvironment. The chimeric antigen receptor of the present invention can recognize tumor cells more specifically and have better targetability to tumor microenvironment.

To achieve the purpose, the present invention adopts the following technical solutions:
In one aspect, the present invention provides a chimeric antigen receptor of anti-placental-like chondroitin sulfate, mainly comprising an antigen recognition region of anti-pl-CS, a hinge region, a transmembrane region, and an intracellular region in tandem arrangement;
Wherein, the antigen recognition region of anti-pl-CS is any one of *Plasmodium falciparum* protein VAR2CSA, partial peptide fragment of the *Plasmodium falciparum* protein VAR2CSA or pl-CS antibody;
The partial peptide fragment of the *Plasmodium falciparum* protein VAR2CSA is a peptide fragment having a number of amino acids greater than 500 from the *Plasmodium falciparum* protein VAR2CSA.

In the present invention, the partial peptide fragment of the *Plasmodium falciparum* protein VAR2CSA refers to a peptide fragment having a number of amino acids greater than 500 from the *Plasmodium falciparum* protein VAR2CSA, and may be any peptide having a number of amino acids of 500 or more than 500 from the *Plasmodium falciparum* protein VAR2CSA, all of which are available. In the present invention, the *Plasmodium falciparum* protein VAR2CSA, the partial peptide fragment ID1-ID2a of the *Plasmodium falciparum* protein VAR2CSA and the pl-CS antibody can specifically bind to pl-CS. Pl-CS is present on almost all types of tumor cells, while not on the surface of normal cells. By selecting pl-CS as the specific target for tumors, using VAR2CSA as the recognition domain of the CAR to recognize pl-CS on tumors, that is, recognizing pl-CS by VAR2CSA, CAR-T cells can be enabled to recognize tumor cells specifically, target the tumor microenvironment more easily and enter into the tumor tissues without damaging normal cells and tissues. In addition, pl-CS is a component of the extracellular matrix of tumors and acts as the material basis for forming tumor microenvironment. Therefore, selecting pl-CS as a target is helpful for CAR-T cells to break through the tumor immunosuppressive microenvironment.

Preferably, the *Plasmodium falciparum* protein VAR2CSA is a VAR2CSA protein of *Plasmodium falciparum* pf 3D7 and/or a VAR2CSA protein of *Plasmodium falciparum* pf FCR3.

Preferably, the VAR2CSA protein of *Plasmodium falciparum* pf 3D7 has an amino acid sequence of SEQ ID NO.3, and a nucleic acid sequence of SEQ ID NO.4.

Preferably, the VAR2CSA protein of *Plasmodium falciparum* pf FCR3 has an amino acid sequence of SEQ ID NO.5, and a nucleic acid sequence of SEQ ID NO.6.

Preferably, the partial peptide fragment of the *Plasmodium falciparum* protein VAR2CSA is ID1-ID2a, which has an amino acid sequence of SEQ ID NO.7, and a nucleic acid sequence of SEQ ID NO.8.

In the present invention, the ID1-ID2a is a peptide with a smaller length and the best effect, among these partial peptide fragments.

Preferably, the hinge region is the hinge region of human CD8α.

Preferably, the hinge region of human CD8α has an amino acid sequence of SEQ ID NO.9, and a nucleic acid sequence of SEQ ID NO.10.

Preferably, the transmembrane region is the transmembrane region of human CD28.

Preferably, the transmembrane region of human CD28 has an amino acid sequence of SEQ ID NO.11, and a nucleic acid sequence of SEQ ID NO.12.

As a preferred technical solution, the intracellular region is any one of the intracellular region of human CD3ζ, the intracellular region of human CD28 or the intracellular region of human 4-1BB, or a combination of at least two thereof, for example, the intracellular region of CD3ζ, the intracellular region of human CD28, the intracellular region of human 4-1BB, the tandem arrangement of intracellular region of human CD28 and intracellular region of human CD3ζ, the tandem arrangement of intracellular region of human 4-1BB and intracellular region of human CD3ζ, the tandem arrangement of intracellular region of human CD3ζ and intracellular region of human 4-1BB, or the tandem arrangement of intracellular region of human CD28, intracellular region of human 4-1BB and intracellular region of human CD3ζ, preferably the intracellular region of CD3ζ, the tandem arrangement of intracellular region of human CD28 and intracellular region of human CD3ζ, the tandem arrangement of intracellular region of human 4-1BB and intracellular region of human CD3ζ, or the tandem arrangement of intracellular region of human CD28, intracellular region of human 4-1BB and intracellular region of human CD3ζ.

Preferably, the intracellular region of human CD3ζ has an amino acid sequence of SEQ ID NO.13, and a nucleic acid sequence of SEQ ID NO.14.

Preferably, the tandem arrangement of intracellular region of human CD28 and intracellular region of human CD3ζ has an amino acid sequence of SEQ ID NO.15, and a nucleic acid sequence of SEQ ID NO.16.

Preferably, the tandem arrangement of intracellular region of human 4-1BB and intracellular region of human CD3ζ has an amino acid sequence of SEQ ID NO.17, and a nucleic acid sequence of SEQ ID NO.18.

Preferably, the tandem arrangement of intracellular region of human CD28, intracellular region of human 4-1BB and intracellular region of human CD3ζ has an amino acid sequence of SEQ ID NO.19, and a nucleic acid sequence of SEQ ID NO.20.

Preferably, the chimeric antigen receptor contains a human CD8α signal peptide at its amino terminus.

Preferably, the human CD8α signal peptide has an amino acid sequence of SEQ ID NO.21 and a nucleic acid sequence of SEQ ID NO.22.

As a preferred technical solution, the chimeric antigen receptor is composed of human CD8α signal peptide, VAR2CSA, hinge region of human CD8α, transmembrane region and intracellular region of human CD28, intracellular region of human 4-1BB and intracellular region of human CD3ζ in tandem arrangement.

Preferably, the chimeric antigen receptor has an amino acid sequence of SEQ ID NO.1 and a nucleic acid sequence of SEQ ID NO.2.

Preferably, the chimeric antigen receptor is expressed through transfecting the nucleic acid sequence encoding it into T cells.

Preferably, the transfection is performed by any one of viral vector, eukaryotic expression plasmid or mRNA sequence, or combination of at least two thereof.

Preferably, the viral vector is any one of lentiviral vector, adenovirus vector or retrovirus vector, or a combination of at least two thereof.

In a second aspect, the present invention provides a composition comprising the chimeric antigen receptor described in the first aspect.

In a third aspect, the present invention provides the use of the chimeric antigen receptor as described in the first aspect and/or the composition as described in the second aspect in preparation of chimeric antigen receptor T cells and in drugs for treating tumors.

Compared with the prior art, the present invention has the following beneficial effects:
(1) Through a manner of recognizing pl-CS by VAR2CSA, the chimeric antigen receptor of the present invention allows CAR-T cells to specifically recognize tumor cells, target the tumor microenvironment more easily, enter into the tumor tissues, recognize tumor cells more specifically and have better targetability to tumor microenvironment;
(2) T cells expressing the chimeric antigen receptor of the present invention can kill tumor cells to the maximum extent, having little damage to normal tissues, and can break through the tumor immunosuppressive microenvironment, allowing them to have a better therapeutic effect on solid tumors and be able to treat almost all types of tumors.

### Description of the Drawings

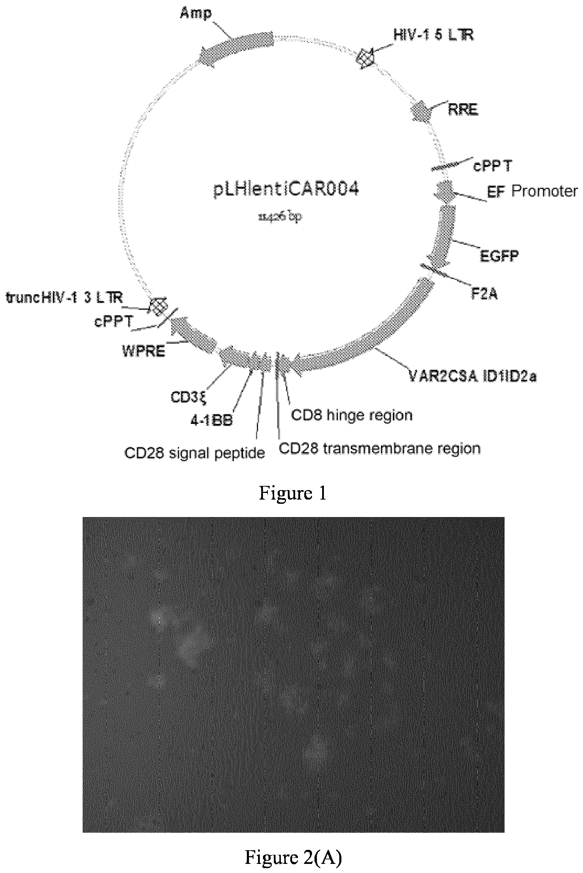

FIG. 1 is a schematic view of a CAR lentiviral expression vector targeting pl-CS;

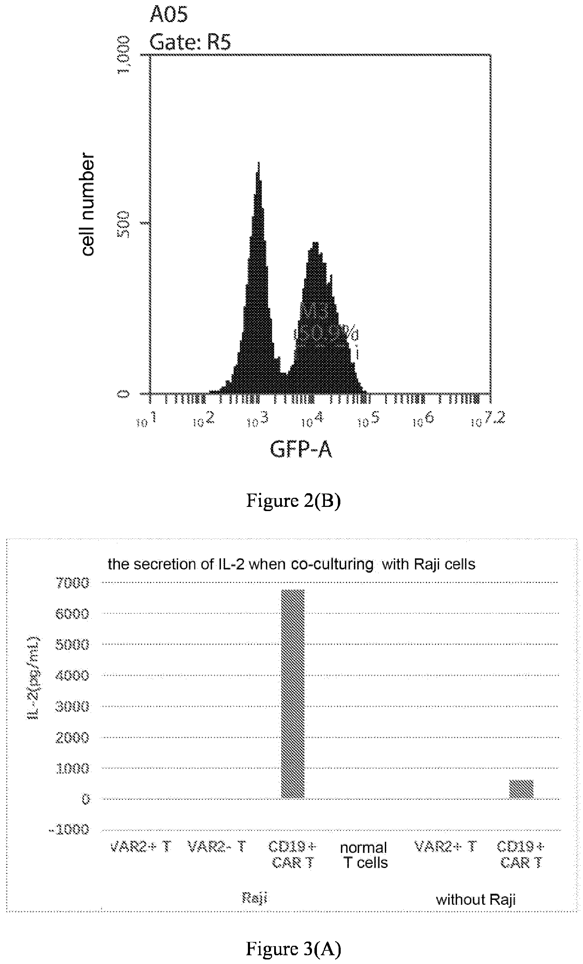

Fig. 2(A) shows the green fluorescence (GFP) expression in T cells transfected with Lentivirus for 96 hours as observed under fluorescence microscope, and Fig. 2(B) shows the GFP positive rate of T cells transfected with Lentivirus for 96 hours as demonstrated by flow cytometry;

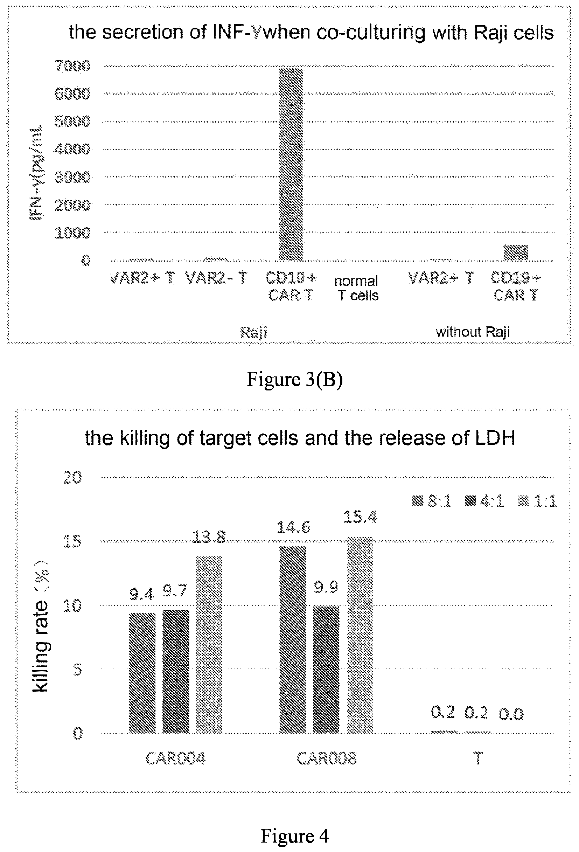

Fig. 3(A) shows a comparison of IL-2 concentrations in the supernatants of the experimental group, the negative control group, the positive control group and the wild type T cell group, and Fig. 3(B) shows the comparison of IFN-γ concentrations in the supernatants of the experimental group, the negative control group, the positive control group and the wild type T cell group;
Figure 4 shows a comparison of the cytotoxicities of the experimental group, the negative control group, and the wild type T cell group against B cell lymphoma cell line Raji under different ratios of effector cells to target cells, wherein the histograms of the experimental group, the negative control group and the wild group are shown from left to right with ratios of effector cells to target cells of 8:1, 4:1, and 1:1, respectively.

### Detailed Description

In order to further illustrate the technical measures adopted by the present invention and the effects thereof, the technical solutions of the present invention are further described below with reference to the accompanying drawings and specific embodiments, and however, the present invention is not limited to the scope of the embodiments.

### Example 1: Determination of the gene sequence of CAR targeting pl-CS

The sequences of the VAR2CSA gene of *Plasmodium falciparum,* the signal peptide gene of human CD8α, the hinge region gene of human CD8α, the transmembrane region and intracellular region genes of human CD28, the intracellular region gene of human 4-1BB, and the intracellular region gene of human CD3ζ were retrieved from the GenBank database and literatures. Codon optimization was performed on these genes to ensure that they were more suitable for expressing in human cells without changing the encoded amino acid sequences.

The sequence information of each gene can be seen from SEQUENCE LISTING (SEQ ID NO.1-22 in the Sequence Listing).

### Example 2: Construction of lentivirus expression vector pLHlentiCAR004

The above gene sequences were sequentially linked in the order of signal peptide gene of human CD8α, VAR2CSA, hinge region gene of human CD8α, transmembrane region and intracellular region genes of human CD28, intracellular region gene of human 4-1BB and intracellular region gene of human CD3ζ to compose the chimeric antigen receptor. The chimeric antigen receptor had a nucleic acid sequence as shown in SEQ ID NO.2. Appropriate restriction enzyme sites were introduced to perform artificial synthesis of the whole gene, which was then cloned into a lentiviral expression vector to obtain pLHlentiCAR004 plasmid. The schematic view of the plasmid was shown in Figure 1.

The recombinant plasmid was sent to Shanghai Yingjun Biotechnology Co., Ltd. for sequencing. The sequencing result was aligned with the sequence intended to be synthesized, demonstrating that the artificially synthesized sequence was completely correct.

### Example 3: Large-scale extraction of lentiviral expression vectors and packaging vectors

The stbl3 *E. coli* harboring pLHlentiCAR004 plasmid and packaging plasmids psPAX2 and pMD2.G was cultured on a large scale in LB medium, and plasmids were extracted on a large scale by alkaline lysis for use in transfection. The specific process was as follows:
1) The stbl3 *E. coli* strains harboring pLHlentiCAR004, psPAX2, or pMD2.G were inoculated into culture flasks containing 500 mL LB/antibiotic culture medium, respectively, and cultured at 37°C on a shaker for 12-16 hours;
2) Bacteria were collected by centrifugation at 3000*g for 10 min. The culture medium was discarded and the culture flask was inverted onto absorbent papers and snapped slightly to allow the residual liquid to be absorbed completely. MaxPure Plasmid Maxi Kit from Guangzhou MK Biotech Co., Ltd. was used to extract plasmids on a large scale from the collected bacteria. The specific steps for plasmid extraction were as follows:
   (1) 20 mL of Buffer P1/RNase A was added to the bacteria, and the flask was vortexed to re-suspend the bacteria (to ensure that the bacteria were completely re-suspended and the re-suspended solution had no cell aggregate); 20 mL of Buffer P2 was added to the resuspension solution, and inverted for 10-15 times to mix, then resting for 1-2 min (be sure to mix gently, note that the operation time cannot exceed 4 minutes);
   (2) 20 mL of Buffer E3 was added to the lysate, and inverted immediately for 15-20 times to mix. The mixing process should be gentle and complete. The standard of complete neutralization was: the solution was no longer viscous and the precipitate was dispersed as white. The resultant was centrifuged at 3000*g for 10 min, then the supernatant was transferred into a new centrifuge tube, 1/3 volume of Buffer E4 was added to the supernatant, vortexed and mixed;
   (3) A MaxPure Micro Column was set into a 50 mL centrifuge tube, 15 mL of the mixture was transferred to the column, and centrifuged at 3000*g for 10 min. The effluent was discarded and the column was set back into the collection tube. Next, the remaining solution was transferred into the column and centrifuged at 3000*g for 10 minutes. The effluent was discarded and the column was set back into the collection tube. 20 mL of Buffer E5 was added to the column, and centrifuged at 3000*g for 10 minutes. The effluent was discarded and the column was set back into the collection tube;
   (4) 20 mL of Buffer PW2 (diluted with absolute ethanol) was added to the column, and centrifuged at 3000*g for 10 minutes. The effluent was discarded and the column was set back into the collection tube. 20 mL of Buffer PW2 (diluted with absolute ethanol) was added to the column, and centrifuged at 3000*g for 10 minutes. The effluent was discarded and the column was set back into the collection tube, and was centrifuged at 4000*g for 10 minutes.
   (5) The column was set into a sterile 50 mL centrifuge tube, and 30-200 mL of sterile water was added to the center of the column membrane, resting for 2 minutes. By centrifugation at 10000 *g for 5 min, the separated solution was collected;
   (6) Further extraction was performed to remove endotoxin, and the plasmid concentration was adjusted with sterile water to be in the range of 0.1g-0.6mg/mL;
   (7) 0.1 volume of Buffer ER1 and 0.1 volume of Buffer ER2 were added to the plasmid solution, and were inverted to mix. Then the mixture was placed on ice for 20min, during which it was inverted and mixed for several times. Next, it was placed in 42 °C water bath for 5min, and then centrifuged at 10000*g at room temperature for 3min;
   (8) The sample was taken out gently and a layer of red solution was found to form at the bottom. The supernatant was carefully transferred into a centrifuge tube, 0.7 volume of isopropyl alcohol was added to the supernatant, and inverted for several times to mix. The mixture was centrifuged at a speed of 10000*g at room temperature for 10min, and the supernatant was discarded. 1 volume of 70% ethanol was added to the precipitate, vortexed and mixed for 15-30 seconds. The resulting mixture was centrifuged at 10000*g at room temperature for 3 minutes, and the supernatant was carefully discarded. Centrifuged at 10000*g at room temperature for additional 1 minute to collect droplets from the tube wall. Do not aspirate the precipitate. Dry in the air for 10 min;
   (9) An appropriate amount of sterile water was added to the plasmid, vortexed for 10-20 seconds, and placed at room temperature for 30 minutes to allow the plasmid to fully dissolve, during which the mixture was inverted and mixed for several times;

The plasmid concentration was measured by a Nanodrop UV spectrophotometer and the plasmid was then stored at -20°C.

### Example 4: Packaging, Concentration and Titration of Lentivirus

### 1) Packaging of Lentivirus

(1) Pre-plating of cells: 24 hours before transfection, 293T cells that were overgrown were pre-plated so that 70% confluence could be reached next day (pre-plating 5 million cells in a 75mm dish).
(2) Day 2: Transfection
   (a) The pre-plated cells were observed for growth. When the cells grew to about 90% confluence, the medium was discarded and freshly pre-warmed complete culture medium was added (15 mL in a 75 mm dish);
   (b) PEI, plasmid and Opti-MEM I Reduced Serum Medium, etc. required for transfection were placed at room temperature for balancing prior to transfection;
   (c) Preparation of mixture of plasmids: A total 10 dishes of 293T cells were packaged this time. 500 µL of mixture should be prepared for each dish, and thus a total volume of 5 mL of mixture should be prepared. Firstly, three types of plasmid were added respectively according to the usage amount of the individual plasmid, with a total of 420 µ g (pLHlentiCAR004 : psPAX2 : pMD2.G=3 : 2 : 1), and Opti-MEM I Reduced Serum Medium was added to adjust the volume to 2.5 mL, mixed by blowing, and placed at room temperature for 10min. Then 1.25 mL of PEI and 1.25 mL of Opti-MEM I Reduced of Serum Medium were mixed and placed at room temperature for 10min. Finally, the above two mixture solutions were mixed and placed at room temperature for 10min to obtain a mixture used for transfection;
   (d) The mixture was added dropwise to the culture dish at 500 µL per dish;
(3) After transfection for 6 h, the medium in each dish was changed to 17 mL of UltraCLUTURE serum-free medium;
(4) Viruses were harvested between Day 3 and Day 5: after transfection for 48h-72h, the culture supernatants were collected respectively. The collected viral supernatants were temporarily stored at 4°C.

### 2) Concentration of Lentivirus

The collected culture supernatants were filtered through a 0.45 um membrane, followed by centrifuging with a SW-32Ti rotor at 25000 rpm (corresponding to a centrifugal force of 106750 g) at 4°C for 2 h. After removing the supernatants, the pellets were re-suspended with 200 µl of PBS, and stored at -80°C.

### 3) Titration of lentivirus

(1) 293T cells were pre-plated into 6-well plates, 3×10⁵ cells/well, and incubated at 37°C in a 5% CO₂ incubator overnight (for approximately 18-20 h);
(2) Preparation of a stock solution of polybrene: sterilized pure water, 8 mg/mL, filtered with a 0.22 µm filter tip, sub-packaged and stored at -20°C for later use.
(3) Preparation of polybrene-containing DMEM medium: 10% FBS, 1% double antibody, 8 µg/mL polybrene (one thousandth of the volume of the medium);
(4) Thawing of viruses: The virus frozen stock was taken out from -80°C, and thawed at room temperature. The thawed viruses were placed on ice and taken into the cytology lab for use.
(5) Dilution of virus: The virus stock solution was diluted 10-fold with 5% FBS DMEM complete culture medium containing polybrene to obtain a diluted virus solution of 10⁻¹-10⁻⁴. Note that the virus solution should be mixed uniformly before taking the solution every time for a further dilution.
(6) The original medium was discarded. 1 mL of virus-free medium was added to the first well as a negative control, and 1 mL of the corresponding virus dilution was added to each of the remaining wells and incubated at 37°C for 18-20 h;
(7) The virus-containing medium was removed the next morning and replaced with 2 mL of 5% FBS DMEM medium without polybrene. The cells were observed under a microscope for the expression of GFP;
(8) The medium was aspirated, and then the cells were digested with trypsin. The cells were blown into single cells, and an appropriate amount of complete culture medium was added to stop the trypsin reaction. The cells were collected by centrifugation at 300 g for 3 min;
(9) The supernatant was decanted, the pellet was re-suspended with an appropriate amount of pre-cooled PBS, followed by centrifugation at 300g for 3 min, and the cells was collected. The above procedure was repeated for one more time;
(10) Finally, the pellet was re-suspended with 1 mL of pre-cooled PBS and the suspension was placed on ice for flow cytometry analysis.
(11) The wells with a GFP positive rate of 1%-20% were selected, and the virus titer of each well was calculated, and finally averaged;
   calculation formula: titer={(F×Cn)/V}×DF, with a unit of TU/ mL;
   wherein: F: the rate of GFP positive cells; Cn: number of cells plated per well; V: volume of virus dilution added; DF: virus dilution factor

The virus titer was calculated to be in the range of: 1.6 × 10⁸-1 × 10¹⁰.

### Example 5: Isolation, culture and infection of T cells

1) Isolation of PBMC cells
   (1) 50 mL of blood was collected and centrifuged at 800 g for 10 min (acceleration was set at 3, speed reduction was set at 4);
   (2) Leukocyte layer was taken and diluted to 8mL with 2% FBS;
   (3) 4mL LymphoPrep was added into a 15mL centrifuge tube, then the diluted blood was carefully added onto the stratification solution along the tube wall to keep a clear interface between the two solutions;
   (4) Centrifuged at 800g for 20min (acceleration was set at 6, speed reduction was set at 1);
   (5) The mononuclear cells with a color of gray white were gently aspirated by using a Pasteur pipette, added to another centrifuge tube already containing 10 mL RF-10, and mixed uniformly;
   (6) Centrifuged at 500 g for 5 min and the supernatant was discarded;
   (7) The cells were re-suspended with 10 mL of RF-10, counted by trypan blue, and centrifuged at 350 g for 10 min, and the supernatant was discarded.
2) Magnetic bead sorting of CD4+T (CD8+T) cells
   (1) Magnetic beads were vortexed and mixed and 25 µL of beads were taken into a test tube. 1 mL of Buffer 1 was added and mixed. Then the test tube was placed on magnetic rack for 1 min. The supernatant was discarded. 25µL of Bffer1 was added to re-suspend the magnetic beads for use;
   (2) PBMCs were re-suspended with Buffer 1 to a density of 10⁷cells/mL;
   (3) 25 µL of the washed magnetic beads was added to 1mL of PBMCs and incubated at 2-8°C for 20 min, by rotating obliquely on a shaking bed;
   (4) The test tube was placed on a magnet for 2 min and the supernatant was collected;
   (5) The test tube was removed, 1 mL of Buffer 1 was added, mixed by blowing, placed on the magnetic beads for 2 min and the supernatant was collected, all of which were repeated once;
   (6) 100 µL of Buffer 2 was added to re-suspend the cells, 10 µL of DETCHaBEAD was added, and incubated at room temperature for 45 min to allow the cells to release from the magnetic beads;
   (7) The test tube was placed on the magnetic rack for 1 min and the supernatant containing cells was transferred to a new test tube. 500 µL of Buffer 2 was added to wash the beads for 2-3 times, and the supernatant was collected;
   (8) 4mL of Buffer 2 was added and centrifuged at 350g for 5min, the supernatant was discarded, and the cells were re-suspended with Buffer 2;
   (9) CD8+ T cells were collected from the supernatant collected during the sorting of CD4+ T cells according to the instruction of kit.
3) Culture of T cells
   (1) CD4+T (CD8+T) cells sorted by magnetic beads were centrifuged at 350g for 10 min;
   (2) The cells were re-suspended with RF-10 and counted;
   (3) CD4+T and CD8+T cells were added to culture plates at a ratio of 1:1 for culture, with a cell density of 5×10⁵ cells/mL;
   (4) CD3/CD28 antibody magnetic beads were added to the T-cell specialized medium at a ratio of magnetic beads to cells of 1:1;
   (5) rhIL-2 was added at a final concentration of 10 ng/mL;
   (6) Cells were counted 2-3 times per week and were recorded for the proliferation.
4) Lentivirus transfection of T cells.
   (1) CD4+T (CD8+T) cells sorted by magnetic beads were centrifuged at 350g for 10 min. The cell pellets were re-suspended in complete culture medium before cell counting.
   (2) CD4+T and CD8+T cells were added to 96-well plates at a ratio of 1:1 for culture, with a cell density of 5×10⁵ cells/mL, and with cell number per well of 1×10⁵;
   (3) The magnetic beads were washed before adding to culture dish at a bead-to-cell ratio of 1:1;
   (4) rhIL-2 was added at a final concentration of 10 µg/L;
   (5) After 24 hours of stimulation, the cells were infected, and polybrene was added at a final concentration of 6 µg/mL, and mixed;
   (6) The medium was changed after 16-24 hours;
   (7) The cells were imaged with a fluorescence microscope three days later.

The result of fluorescence microscopy was shown in Fig. 2(A) and the infection efficiency detected by flow-cytometry was shown in Fig. 2(B). It can be seen that the infection efficiency detected by flow-cytometry was about 50%, and CAR-T cells can be efficiently produced.

### Example 6: In vitro release and detection of cytokines of CAR-T cells

(1) In a 48-well plate, CAR-T cells, 1.5×10⁵ per well, were co-cultured with Huh7 cells, 1.5×10⁵ cells per well, for 24 hours, and 500 µL of RPMI-1640 culture medium (without serum and rhIL-2) was added;
(2) Collection and storage of samples: the supernatant was collected by centrifugation, and used for experiment directly or sub-packaged and stored at -20°C (100 µL per tube);
(3) Preparation of reagents: all the reagents were taken out from storage and allow them to warm to room temperature. Wash Buffer concentrate was diluted to 500 mL with deionized water. A and B solutions were mixed in equal volumes to give a color-substrate solution, which should be used within 15 minutes, and at an amount of 200 µL per well. For cytokine standards, 500 µL stock solution was taken and diluted into 6 concentrations in gradient;
(4) Samples and the gradiently diluted standards were respectively added to ELISA plates which had been coated with antibody at an amount of 100µL per well, removing air bubbles, and incubated at room temperature for 2h (three replicates were set for each group of samples);
(5) Fluid in the wells was removed, 300 µL of WB was added into each well for washing, the washing was repeated for three times, and the WB was completely aspirated at the last washing;
(6) 200 µL of corresponding detection antibodies were added and incubated for 2 h at room temperature;
(7) Fluid in the wells was removed, 300 µL of WB was added into each well for washing, the washing was repeated for three times, and the WB was completely aspirated at the last washing;
(8) 200 µL of substrate solution was added and incubated at room temperature for 20 min (dark light);
(9) 50 µL of stop solution was added. The color of the solution was changed from blue to yellow. If the color is green or doesn't change, tap slightly to mix the solution;
(10) Absorbance was measured at 450 nm within 30min;
(11) Concentrations of various cytokines were calculated according to standard curves,

The results were shown in Figure 3 (A) and 3 (B), from which it can be seen that CAR-T cells do not release cytokines when co-culturing with tumor cells, suggesting that the CAR-T cells have no adverse effect of strong cytokine storms, and thus will not cause side effects such as inflammatory reactions.

### Example 7: In vitro killing tumor cells by CAR-T cells

1) Determination of the optimal cell-plating concentrations
   (1) Various tumor cells were collected, washed with assay medium, and adjusted to a concentration of 1×10⁵ cells/mL;
   (2) 100 µL of assay medium was added to 96-well plate and bespread;
   (3) 100 µL of cell suspension was added to the 96-well plate and serial dilution was performed. 100 µL of cell suspension was aspirated from the final concentration gradient. Six duplicate wells were set with three wells being High control group and three wells being Low control group, and three wells containing assay medium without cells were set as Background control (range, 500-50000);
   (4) Incubated in an incubator for 18 hours;
   (5) 5 µL of cell lysate was added to each well of the HC group and incubated for 15 min. Shook to accelerate cell lysis.
   (6) 100 µL of freshly prepared reaction solution was added to each well and incubated at 15°C-25°C for 30 minutes (note that the 96-well plate should be protected from light);
   (7) 50 µL of stop solution was added to each well and shook for 10 s;
   (8) Absorbance was measured at 490 nm;
   (9) The data was analyzed to determine the optimal cell concentration (maximum difference between HC and LC).
2) Detection of cell-mediated toxicity
   (1) CAR-T cells were collected and washed with assay medium;
   (2) The number of CAR-T cells to be plated in a 96-well plate was determined according to different ratios of effector cells to target cells and the optimal concentrations as the results in 1);
   (3) The corresponding cell suspension to be plated in the 96-well plate was adjusted to 50 µL volume per well.
   (4) Various tumor cells were collected and the cell concentrations were adjusted to 2 times of the optimal concentrations. 50 µL of cell suspension was added into T cells;
   (5) Different control groups were set up;
   (6) Incubated in an incubator at 37°C for 18h;
   (7) 5 µL of cell lysate was added to each well and incubated for 15 min. Shook to accelerate cell lysis.
   (8) 100 µL of freshly prepared reaction solution was added to each well and incubated at 15°C-25°C for 30 minutes (note that the 96-well plate should be protected from light);
   (9) 50 µL of stop solution was added to each well and shook for 10 s;
   (10) Absorbance was measured at 490 nm;
   (11) Each sample was calculated for the percentage of cytotoxicity.

The results were shown in Figure 4. Under different ratios of effector cells to target cells, CAR-T cells have toxicity on tumor cells, and can efficiently kill tumor cells and release LDH. However, wild type T cells, as a control group, cannot kill tumor cells.

In summary, T cells expressing the chimeric antigen receptor of the present invention can kill tumor cells to the maximum extent, without releasing of cytokines, having little damage to normal tissues, and can break through the tumor immunosuppressive microenvironment, allowing them to have a better therapeutic effect on solid tumors and be able to treat almost all types of tumors.

The applicant states that detailed methods of the present invention are demonstrate in the present invention through the above embodiments, however, the present invention is not limited to the above detailed methods, and does not mean that the present invention must rely on the above detailed methods to implement. It should be apparent to those skilled in the art that, for any improvement of the present invention, the equivalent replacement of the raw materials of the present invention, the addition of auxiliary components, and the selection of specific modes, etc., will all fall within the protection scope and the disclosure scope of the present invention.

## Claims

1. A chimeric antigen receptor of anti-placental-like chondroitin sulfate, mainly comprising an antigen recognition region of anti-pl-CS, a hinge region, a transmembrane region, and an intracellular region in tandem arrangement;
wherein, the antigen recognition region of anti-pl-CS is any one of *Plasmodium falciparum* protein VAR2CSA, partial peptide fragment of the *Plasmodium falciparum* protein VAR2CSA, or antibody against pl-CS; and
the partial peptide fragment of the *Plasmodium falciparum* protein VAR2CSA is a peptide fragment having a number of amino acids greater than 500 from the *Plasmodium falciparum* protein VAR2CSA.

2. The chimeric antigen receptor according to claim 1, wherein the *Plasmodium falciparum* protein VAR2CSA is a VAR2CSA protein of *Plasmodium falciparum* strain pf 3D7 and/or a VAR2CSA protein of *Plasmodium falciparum* strain pf FCR3;
preferably, the VAR2CSA protein of *Plasmodium falciparum* strain pf 3D7 has an amino acid sequence of SEQ ID NO.3, and a nucleic acid sequence of SEQ ID NO.4;
preferably, the VAR2CSA protein of *Plasmodium falciparum* strain pf FCR3 has an amino acid sequence of SEQ ID NO.5, and a nucleic acid sequence of SEQ ID NO.6;
preferably, the partial peptide fragment of the *Plasmodium falciparum* protein VAR2CSA is ID1-ID2a, which has an amino acid sequence of SEQ ID NO.7, and a nucleic acid sequence of SEQ ID NO.8.

3. The chimeric antigen receptor according to claim 1 or 2, wherein the hinge region is the hinge region of human CD8α;
preferably, the hinge region of human CD8α has an amino acid sequence of SEQ ID NO.9, and a nucleic acid sequence of SEQ ID NO.10.

4. The chimeric antigen receptor according to any one of claims 1 to 3, wherein the transmembrane region is the transmembrane region of human CD28;
preferably, the transmembrane region of human CD28 has an amino acid sequence of SEQ ID NO.11, and a nucleic acid sequence of SEQ ID NO.12.

5. The chimeric antigen receptor according to any one of claims 1 to 4, wherein the intracellular region is any one of the intracellular region of human CD3ζ, the intracellular region of human CD28, or the intracellular region of human 4-1BB, or a combination of at least two thereof, and preferably is the intracellular region of CD3ζ, the tandem arrangement of the intracellular region of human CD28 and the intracellular region of human CD3ζ, the tandem arrangement of the intracellular region of human 4-1BB and the intracellular region of human CD3ζ, or the tandem arrangement of the intracellular region of human CD28, the intracellular region of human 4-1BB and the intracellular region of human CD3ζ;
preferably, the intracellular region of human CD3ζ has an amino acid sequence of SEQ ID NO.13, and a nucleic acid sequence of SEQ ID NO.14;
preferably, the tandem arrangement of the intracellular region of human CD28 and the intracellular region of human CD3ζ has an amino acid sequence of SEQ ID NO.15, and a nucleic acid sequence of SEQ ID NO.16;
preferably, the tandem arrangement of the intracellular region of human 4-1BB and the intracellular region of human CD3ζ has an amino acid sequence of SEQ ID NO.17, and a nucleic acid sequence of SEQ ID NO.18;
preferably, the tandem arrangement of the intracellular region of human CD28, the intracellular region of human 4-1BB and the intracellular region of human CD3ζ has an amino acid sequence of SEQ ID NO.19, and a nucleic acid sequence of SEQ ID NO.20.

6. The chimeric antigen receptor according to any one of claims 1 to 5, wherein the chimeric antigen receptor further contains a human CD8α signal peptide at its amino terminus;
preferably, the human CD8α signal peptide has an amino acid sequence of SEQ ID NO.21 and a nucleic acid sequence of SEQ ID NO.22.

7. The chimeric antigen receptor according to any one of claims 1 to 6, wherein the chimeric antigen receptor is composed of human CD8α signal peptide, VAR2CSA, hinge region of human CD8α, transmembrane region and intracellular region of human CD28, intracellular region of human 4-1BB and intracellular region of human CD3ζ in tandem arrangement;
preferably, the chimeric antigen receptor has an amino acid sequence of SEQ ID NO.1 and a nucleic acid sequence of SEQ ID NO.2.

8. The chimeric antigen receptor according to any one of claims 1 to 7, wherein the chimeric antigen receptor is expressed through transfecting the nucleic acid sequence encoding it into T cells;
preferably, the transfection is performed by any one of viral vector, eukaryotic expression plasmid, or mRNA sequence, or combination of at least two thereof;
preferably, the viral vector is any one of lentiviral vector, adenovirus vector, or retrovirus vector, or a combination of at least two thereof.

9. A composition comprising the chimeric antigen receptor described in any one of claims 1 to 8.

10. Use of the chimeric antigen receptor as described in any one of claims 1 to 8 and/or the composition as described in claim 9 in preparation of chimeric antigen receptor T cells and in drugs for treating tumors.
